# EUROPEAN PATENT SPECIFICATION

(11) **EP 3 493 759 B1**
(45) Date of publication and mention of the grant of the patent: **07.10.2026**
(21) Application number: 17838216.4
(22) Date of filing: 09.08.2017
(51) Int. Cl.: A61B 18/20

(54) **ROBOT-ASSISTED LASER SURGICAL SYSTEM**
ROBOTERGESTÜTZTES LASERCHIRURGISCHES SYSTEM
SYSTÈME CHIRURGICAL LASER ASSISTÉ PAR ROBOT

(30) Priority: 10.08.2016 AU 2016903144
(43) Date of publication of application: 12.06.2019
(73) Proprietor: Australian Institute of Robotic Orthopaedics Pty Ltd, Nedlands WA 6009 (AU)
(72) Inventor: KHAN, Riaz Jan Kjell, Cottesloe, Western Australia 6011 (AU); FICK, Daniel Paul, Cottesloe, Western Australia 6011 (AU); ROBERTSON, William Brett, North Fremantle, Western Australia 6159 (AU); SHEH, Raymond Ka-Man, Bentley, Western Australia 6102 (AU); IRONSIDE, Charles, South Perth, Western Australia 6151 (AU); CHIPPER, Richard, Mount Claremont, Western Australia 6010 (AU)
(74) Representative: Manitz Finsterwald Patent- und Rechtsanwaltspartnerschaft mbB
(86) International application number: PCT/AU2017/050840
(87) International publication number: WO 2018/027269

(56) References cited:
- WO-A1-2014/102355
- WO-A1-2014/102355
- US-A1- 2012 220 992
- US-A1- 2012 220 992
- US-B2- 7 831 292
- US-B2- 7 831 292
- BURGNER ET AL: "Robot assisted laser osteotomy", 1 January 2010 (2010-01-01), XP055463769, Retrieved from the Internet <URL:https://www.ksp.kit.edu/9783866444973> [retrieved on 20180329]
- JOHANNES J. KUTTENBERGER ET AL: "Computer-Guided CO 2 -Laser Osteotomy of the Sheep Tibia: Technical Prerequisites and First Results", PHOTOMEDICINE AND LASER SURGERY, vol. 26, no. 2, 1 April 2008 (2008-04-01), US, pages 129 - 136, XP055463774, ISSN: 1549-5418, DOI: 10.1089/pho.2007.2139
- BURGNER, J.: "Robot assisted laser osteotomy", 2014, XP055463769, Retrieved from the Internet <URL:https://www.ksp.kit.edu/9783866444973>
- KUTTENBERGER, JOHANNES J. ET AL.: "Computer-guided C02- laser osteotomy of the sheep tibia: technical prerequisites and first results", PHOTOMEDICINE AND LASER SURGERY, vol. 26, no. 2, 2008, pages 129 - 136, XP055463774

## Description

### TECHNICAL FIELD

The present invention relates generally to performing work in respect of laser shaping of biological tissue including bone. In particular, the invention relates to the field of laser osteotomy systems as known from US 2012 / 220 992 A or WO 2014 / 102 355 A1.

Although the present invention will be described with particular reference to work comprising one or more actions of an orthopaedic surgical procedure performed on dynamic material comprising biological tissue of a living human being, it will be appreciated that implementations of the invention may be used in respect of other biological tissue, and for work comprising additional and/or alternative actions.

### BACKGROUND

Any discussion of the background art throughout the specification should in no way be considered as an admission that such background art is prior art, nor that such background art is widely known or forms part of the common general knowledge in the field in Australia or worldwide.

Biological tissue can be difficult to work.

One type of biological tissue that is difficult to work is bone. From a materials processing point of view, hard biological tissue, such as bone, is an unfavourable material due to its complex physical nature based on multicomponent (calcium phosphate (ceramic), collagen (organic), and water) and hierarchical structure.

Osteotomy is the surgical cutting of bone. This technique is routinely used in orthopaedic surgery, for example during joint arthroplasty in which parts of an arthritic or damaged joint are removed and replaced with one or more components such as an implant/prosthesis intended to replicate movement of a normal, healthy joint.

A cutting process forms one of the important bone shaping operations during orthopaedic surgeries. Successful joint arthroplasty relies on accurate and precise osteotomy to ensure the alignment of the components and the security of fixation to the bone. Accuracy and quality of bone preparation are key factors in long term implant survivability and good clinical outcome.

For example, the outcome for a recipient of Total Knee Replacement (TKR) surgery is largely dependent on the accuracy of the set of resections performed as part of the surgery to match the internal geometry of the prosthetic implants. Each resection can differ from the ideal cut plane in terms of the position, angle, and flatness of the resulting surface. Inaccuracies in the position and angle result in poor function of the prosthetic as well as potential looseness of the implant. Inaccuracies in the flatness of any surface results in "point loading" that can damage the bone and affects the positioning of the implant - decreasing the longevity of the implant and the quality of life of the patient.

As will be described in further detail, current joint surgical techniques lack accuracy in positioning the resections. It has been reported that 20% of total knee replacement patients are dissatisfied with the outcome of the surgery postoperatively and aseptic loosening continues to persist as the leading cause of revision within 10 years of surgery.

Orthopaedic surgery has come a long way through adaptation/integration of modern tools such as sensors and computer aided design (CAD) based generation of patient specific defined joint design and bone machining (shaping/cutting) parameters. That said, currently to perform osteotomy surgeons use conventional mechanical tools such as saws, drills, hammers, ultrasonic cutters, chisels, and grinders. These instruments suffer from a number of disadvantages, including that they lack accuracy and submillimetre precision, cause thermal damage, transfer vibrations and biomechanical stress to adjacent bone, and can cause bone fragmentation.

Particularly, conventional sawing techniques produce uneven bone surfaces with gaps large enough to affect the fixation and position of the implant. Modern surgical instrumentation incorporates various features (such as narrow slots to guide saw blades) that are designed to improve resection accuracy. Nevertheless, making precise bone cuts with current instrumentation in the clinical setting is difficult, and varus-valgus cutting errors of 4 degrees and flexion-extension errors of 10 degrees have been reported. Clinical studies have shown that successful bone ingrowth into porous coating necessitates both close bone apposition to the porous surface and sufficient initial fixation of the implant. Furthermore, the heat generated by mechanical osteotomy, caused by friction along with heavy mechanical loading, can lead to tissue necrosis (death). Tissue necrosis adversely affects the bony integration into the implant reducing long-term implant survivability.

Currently orthopaedic surgeons rely on a combination of mechanical cutting tools used in conjunction with cutting jigs, computer navigation, and patient matched cutting blocks. Whilst these tools are currently widely accepted as gold standard they each have an inherent degree of error. The accumulative impact of this error is multiplied exponentially when additional factors such as surgeon experience and technical competence are accounted for.

Indeed, the conventional way of orthopaedic surgery is associated with human and tool attributes that mostly result in potentially increasing the risk of thermal damage (necrosis). This situation in turn leaves tremendous room for further development of operating tools and techniques. Further developments are needed to address the adverse effects of orthopaedic surgery including, but not limited to, 1) severe damage of tissues within and surrounding repaired/operated regions, 2) low precision in final dimensional tolerance on repaired/operated bone, 3) relatively slow surgical processes, 4) post-surgery tissue trauma, 5) rigorous pain, and 6) in some cases, post-surgery complications requiring further surgery and related addition of cost and 7) low precision in final component positioning.

Cutting saw and osteotomes are the traditional tools employed for bone cutting during orthopaedic surgery. Newer techniques include burrs. Being a manual operation, it involves human errors and necessity of skilled surgeons thus making achievement of reproducibility difficult. Apart from these variabilities, there are other issues associated with their use, such as the above mentioned thermally driven necrosis of tissues initiated by temperature rise due to the extended period of physical contact between the cutting/shaping tool and bone that leads to friction/abrasion between the cutting tool and bone along with heavy mechanical loading of bones during conventional mechanical shaping/cutting. In general, cutting saw blades are much harsher than cutting burrs, with temperatures of bone rising above 100°C. The reason behind this increase in temperature is the large contact area of bone with the teeth of the saw. In addition, there are many cuts that need to be performed in order to shape the bone. Although the cutting operation using a burr results in moderate temperatures (50-60 °C), burr cutting is limited to shallow cuts, and is thus not an ideal substitute for the cutting saw.

To address the temperature rise and avoid associated necrosis, many remedies have been explored that are mainly focused on (a) change in tool design, (b) improving method of operation, and most prevalent (c) employing saline cooling. Out of these, (a), and (b) still require careful operational procedures to achieve lower heat generation. In the case of (c), even though the temperature rise can be controlled, designing an effective cooling system becomes necessary. For cutting tools, internally cooled tools have been reported to be superior in terms of heat control than an external spray/mist cooling. Intricately designed tools and careful temperature and flow rate control are required to achieve low heat generation. Furthermore, due to the physical contact between the mechanical cutting tools and bone, a very cautious sterilization process for tools is required to avoid any risk of infection. Apart from these issues, the conventional bone shaping/cutting also involves post tissue trauma, rigorous pain and long healing/recovery times.

Osteoarthritis (OA) is a leading cause of disability in developed nations. In Australia it afflicts 16.5% of the adult population (3.9 million Australians). Total joint arthroplasty is considered the treatment of choice for end stage osteoarthritis. The number of total joint arthroplasties undertaken in Australia has doubled over the last decade with more than 80,000 procedures performed during 2014 at a cost to the Australian health system of over AUD 1 billion.

According to the World Health Organisation (WHO), by 2050 130 million people will suffer from OA worldwide, of whom 40 million will be severely disabled by the disease.

Compelling demographic trends, such as the growing, aging and more active population and rising obesity rates are expected to be key drivers in the continued growth of OA. According to the United Nations, by 2050 people aged over 60 will account for more than 20% of the world's population. Of that 20%, a conservative estimate of 15% will have symptomatic OA, and one-third of these people will be severely disabled. According to the WHO, it is estimated that in 2014 more than 1.9 billion adults, 18 years and older, were overweight. Of these over 600 million were obese. According to the American Journal of Epidemiology, obese women had nearly four times the risk of suffering from OA of the knee as non-obese women, and obese men had nearly five times the risk of suffering from OA of the knee as non-obese men.

For the most severe cases of OA, in which patients suffer from extreme pain, reconstructive joint surgery may be required. Reconstructive joint surgery involves the removal of the bone area surrounding the affected joint and the insertion of one or more manufactured implants as a replacement for the affected bone. According to Knowledge Enterprises, Inc., the joint replacement product market as a whole, including knees, hips, elbows, wrists, digits and shoulders, is estimated to have approached $14.9 billion worldwide in 2013. According to Frost & Sullivan, the global joint implant market in 2014 was $34.9 billion, with knee and hip implant systems representing the two largest sectors.

It is against this background that the present invention has been developed.

### SUMMARY

The invention relates to a system as defined in the appended claims. Embodiments, examples or aspects in the following disclosure which do not fall under the scope of the claims, in particular methods, are presented for illustration purposes only and do not form part of the invention.

It is an object of the present invention to overcome or ameliorate at least one or more of the disadvantages of the prior art, or to provide a useful alternative.

Throughout this specification, unless the context requires otherwise, the words "comprise", "comprises" and "comprising" will be understood to imply the inclusion of a stated step or element or group of steps or elements but not the exclusion of any other step or element or group of steps or elements.

Any one of the terms: "including" or "which includes" or "that includes" as used herein is also an open term that also means including at least the elements/features that follow the term, but not excluding others. Thus, "including" is synonymous with and means "comprising".

In the claims, as well as in the summary above and the description below, all transitional phrases such as "comprising," "including," "carrying," "having," "containing," "involving," "holding," "composed of," and the like are to be understood to be open-ended, i.e., to mean "including but not limited to". Only the transitional phrases "consisting of" and "consisting essentially of" alone shall be closed or semi-closed transitional phrases, respectively.

The term "real-time", for example, "displaying real-time data," refers to the display of the data without intentional delay, given the processing limitations of the system and the time required to accurately measure the data.

Although any methods and materials similar or equivalent to those described herein can be used in the practice or testing of the present invention, preferred methods and materials are described. It will be appreciated that the methods, apparatus and systems described herein may be implemented in a variety of ways and for a variety of purposes. The description here is by way of example only.

The various methods or processes outlined herein may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other non-transitory medium or tangible computer storage medium) encoded with one or more programs that, when executed on one or more computers or other processors, perform methods that implement the various embodiments of the invention discussed above. The computer readable medium or media can be transportable, such that the program or programs stored thereon can be loaded onto one or more different computers or other processors to implement various aspects of the present invention as discussed above.

The terms "program" or "software" are used herein in a generic sense to refer to any type of computer code or set of computer-executable instructions that can be employed to program a computer or other processor to implement various aspects of embodiments as discussed above. Additionally, it should be appreciated that according to one aspect, one or more computer programs that when executed perform methods of the present invention need not reside on a single computer or processor, but may be distributed in a modular fashion amongst a number of different computers or processors to implement various aspects of the present invention.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

Also, various inventive concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The phrase "and/or", as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of" will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one", in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

For the purpose of this specification, where method steps are described in sequence, the sequence does not necessarily mean that the steps are to be carried out in chronological order in that sequence, unless there is no other logical manner of interpreting the sequence.

In addition, where features or aspects of the invention are described in terms of Markush groups, those skilled in the art will recognise that the invention is also thereby described in terms of any individual member or subgroup of members of the Markush group.

Embodiments of the present invention seek to overcome, or at least ameliorate, one or more of the deficiencies of the prior art mentioned above, or to provide the consumer with a useful or commercial choice.

Other advantages of embodiments of the present invention will become apparent from the following description, taken in connection with the accompanying drawings, wherein, by way of illustration and example, preferred embodiments of the present invention are disclosed.

### BRIEF DESCRIPTION OF THE DRAWINGS

Notwithstanding any other forms which may fall within the scope of the present invention, a preferred embodiment / preferred embodiments of the invention will now be described, by way of example only, with reference to the accompanying drawings in which:
**Figure** 1 depicts a high-level physical system architecture of an embodiment of a system in accordance with aspects of the present invention;
**Figure 2A** depicts a particular embodiment of an end effector of the system of **Figure** 1;
**Figure 2B** depicts an example usage configuration of the end effector of the system of **Figure 1** for shaping bone tissue of a patient during a surgical procedure;
**Figures 3****,** **4** and **5** depict alternative embodiments of an end effector of the system of **Figure 1****;**
**Figure 6** depicts an alternative embodiment of laser transmission to an end effector of the system of **Figure 1****;**
**Figure 7** depicts an embodiment of the sensors and sensor controllers of an end effector of the system of **Figure 1****;**
**Figure 8** depicts resections of the bones for implants of a total knee replacement surgical operation;
**Figure 9** depicts positioning and internal structure of a consumable shield/particle collector of the system of **Figure 1**;
**Figure 10** depicts a base unit of the system of **Figure 1****;**
**Figure 11** depicts a graphical representation of ablation run risk to target and non-target "collateral" tissue;
**Figures 12A** and **12B** depict laser beam ablation dimensions and patterns of the system of **Figure 1****;**
**Figure 13** depicts architecture of a controller of the system of **Figure 1****;**
**Figure 14** depicts a flow chart of actions taken during a watch dog (hardware failsafe) procedure of the system of **Figure 1****;**
**Figure 15** depicts a flow chart of actions performed by a system coordinator of the controller of **Figure 13****;**
**Figure 16** depicts a flow chart of actions performed during a mapping operation of the controller of **Figure 13****;**
**Figure 17** depicts a flow chart of actions performed during a planning operation of the controller of **Figure 13****;**
**Figure 18** depicts a flow chart of actions performed during a landmark registration operation of the planning operation of **Figure 17****;**
**Figure 19** depicts a flow chart of actions performed during a position over material operation of the planning operation of **Figure 17****;**
**Figure 20** depicts a flow chart of actions performed during a position at standby operation of the planning operation of **Figure 17****;**
**Figure 21** depicts a flow chart of actions performed for a set of prosthetic component resections with fit and alignment analysis of the planning operation of **Figure 17****;**
**Figure 22** depicts a flow chart of actions performed during a resection operation of the planning operation of **Figure 21****;**
**Figure 23** depicts a flow chart of actions performed during a resection surface 3D to 2D projection of the resection operation of **Figure 22****;**
**Figure 24** depicts a flow chart of actions performed during a resection surface 2D to 3D projection of the resection operation of **Figure 22****;**
**Figure 25** depicts a flow chart of actions performed during a resection surface analysis operation of the controller of **Figure 1** and operation of **Figure 22****;**
**Figure 26** depicts a flow chart of the actions performed during a prosthetic components' fit and alignment analysis operation of the prosthetic resections of **Figure 21****;**
**Figure 27** depicts a flow chart of actions performed during a bioanalysis operation of the controller of **Figure 13****;**
**Figure 28** depicts a flow chart of actions performed during an acoustic analysis operation of the controller of **Figure 13****;**
**Figure 29** depicts a flow chart of actions performed during a laser optimisation operation of the controller of **Figure 13****;** and
**Figure 30** depicts a computing device on which the various embodiments described herein may be implemented in accordance with an embodiment of the present invention.

### DETAILED DESCRIPTION

The present invention is not to be limited in scope by the following specific embodiments. This detailed description is intended for the purpose of exemplification only.

Further features of the present invention are more fully described in the examples herein. It is to be understood, however, that this detailed description is included solely for the purposes of exemplifying the present invention, and should not be understood in any way as a restriction on the broad description of the invention as set out hereinbefore.

Throughout this specification, unless the context requires otherwise, the word "comprise", or variations such as "comprises" or "comprising", will be understood to imply the inclusion of a stated integer or group of integers but not the exclusion of any other integer or group of integers.

Furthermore, throughout this specification, unless the context requires otherwise, the word "include", or variations such as "includes" or "including", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

The invention described herein may include one or more range of values (for example, size, displacement and field strength etc.). A range of values will be understood to include all values within the range, including the values defining the range, and values adjacent to the range that lead to the same or substantially the same outcome as the values immediately adjacent to that value which defines the boundary to the range. For example, a person skilled in the field will understand that a 10% variation in upper or lower limits of a range can be totally appropriate and is encompassed by the invention. More particularly, the variation in upper or lower limits of a range will be 5% or as is commonly recognised in the art, whichever is greater.

Throughout this specification relative language such as the words 'about' and 'approximately' are used. This language seeks to incorporate at least 10% variability to the specified number or range. That variability may be plus 10% or negative 10% of the particular number specified.

In the drawings, like features have been referenced with like reference numbers.

From the discussion of the background art herein, it is evident that conventional cutting/shaping techniques, in spite of having short comings, are still in use. The embodiment of the invention seeks to improve this situation by aiming at the elimination of (a) complex system arrangements, (b) variabilities introduced by human factors, and (c) physical contact between the cutting/shaping tool and bone.

To this end, the implementation takes a different approach so as to seek a superior shaping/cutting operation. It is based on the employment of an extremely short duration, non-physical contact, high intensity laser beam as an energy source for shaping/cutting. The pulse width (alternatively pulse duration) of laser pulses from laser module **130** may be in the range of between about 0.1 to 1000 µs. The average output power of the laser from laser module **130** may be in the range of between about 0.1 to 2000 W. Such a high intensity focused laser beam may advantageously conduct bone material removal in an extremely short time duration without causing any, or at least reducing, thermal necrosis and mechanical damage to the material surrounding the bone-laser interaction region **63.** The laser beam **30** is focused to a spot on the biological material **12** with a spot size of typically between about 300 and 1000 µm in diameter so as to deliver laser energy to the biological material **12** for ablating the material with laser energy having an energy density (fluence) in the range of between about 0.1 to 1000 J/cm² and, under normal operation, typically in the range of between about 10 to 150 J/cm². Furthermore, as will be described in further detail, the disclosed laser based bone shaping/cutting technique is highly amenable to automation and reduction in human intervention and operation time along with increasing precision in shaping/cutting. In addition, these primary advantages may lead to secondary benefits such as rapid patient recovery and reduction in cost.

Any laser types suitable for bone shaping/cutting operation may be used for example: gas lasers such as a CO₂ laser (having emission wavelength between about 9 µm to about 12 µm); or solid state lasers including: Er:YAG laser (wavelength: 2940 nm), Nd:YAG laser (wavelength: 1064 nm), Tm:YAG laser (wavelength: 2000 nm), Ho:YAG laser (wavelength: 2100 nm) or other suitable laser system as would be appreciated by the skilled addressee, preferably having an emission wavelength compatible with absorption by water which is abundant in biological tissue and which can also be applied to the interaction site to increase the efficiency of the ablation process thorough interaction between the laser radiation **30** and a water-based solution. Water has a significant absorption coefficient around 3 µm so a laser source capable of laser emission about this absorption peak (for example an Er:YAG laser emitting at 2940 nm is particularly suited to laser ablation processes in biological material **12).** It should be appreciated that the proposed laser based shaping is well suited for semi or full automation.

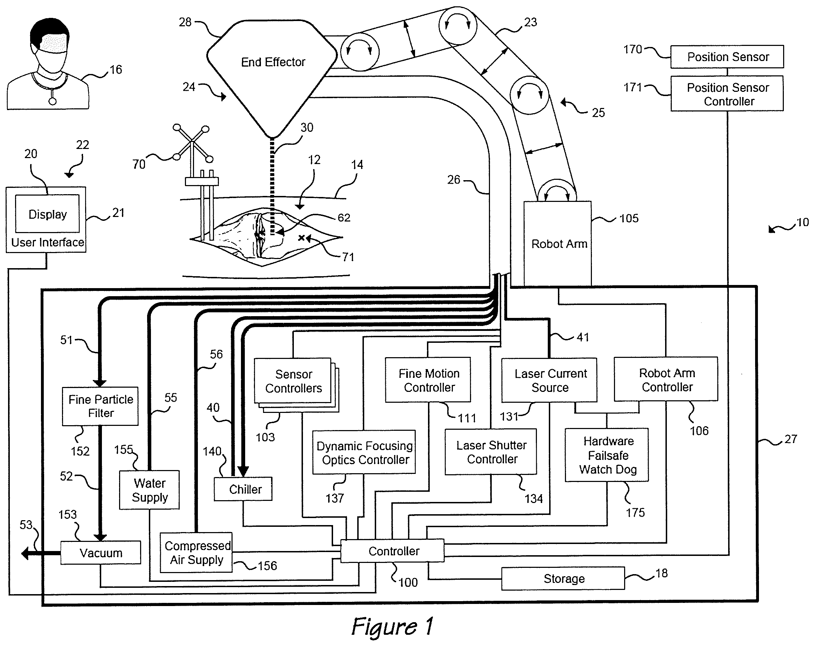

In **Figure 1****,** there is depicted an embodiment of a system **10** for working biological tissue **12** in accordance with aspects of the present invention, illustrated at a high-level as an interconnected collection of physical components.

In the embodiment described, the biological tissue **12** is dynamic material comprising a portion of patient **14** that is a living human being. The work comprises one or more actions of a surgical procedure to be performed on the patient **14.** The biological tissue **12** comprises soft tissue, such as cartilage, and hard tissue, such as bone, of which a subsection is able to be ablated away by the system **10** to result in a resectioned surface, which, for joint arthroplasty, is ideal for the prosthetic implant. The so-called "dynamic" nature of the biological tissue **12** indicates that it may shift or move during the procedure and this needs to be accounted for in the method of ablation to ensure that the surface is accurately targeted, discussed in further detail hereafter.

The system **10** may be referred to as an Intelligent Robot Assisted Subtractive Laser Ablation System (**IRASLAS**^{™}) and, as will be described, is operable to perform one or more resections required for osteotomy, joint arthroplasty, or any other orthopaedic or reconstructive procedure, using computer controlled robotic positioning, precise laser ablation and sensors to assess the process and report on the resulting geometry.

The system **10** comprises a plurality of components, subsystems and/or modules operably coupled via appropriate circuitry, computer chips (integrated circuits), transceiver/receiver antenna, software and connections to enable the system 10 to advantageously combine the precision of computer controlled equipment with the fidelity of a laser in performing the functions and operations herein described.

Particularly, and as shown in **Figure 1****,** the system **10** comprises: a tool **24** operable to perform at least one action of work; positioning means **25** for positioning the tool **24** relative to the biological tissue **12** and a directed laser light beam **30** to perform the at least one action of work required as part of a surgical procedure; computing means which in this embodiment comprises a controller **100** and storage **18** storing electronic program instructions for controlling the controller **100,** and information and/or data; a display **20** for displaying a user interface **21** and input means **22** for the surgeon **16** to interact with the system **100;** and hardware components stored in a base unit **27** that connect to the tool via a conduit **26.**

The position means **25** may comprise any component, or assembly of components, operable to move the tool **24** and/or the biological tissue **12** relative to one another so that the tool **24** can perform the at least one action of work on the biological tissue **12**

In the preferred embodiment, the tool **24** comprises an end effector **28** that is operably attached at the working end of a robot arm **105** comprising the positioning means **25.** The end effector **28** is a hardware assembly comprising several components and sensors, as described in further detail herein, to form and direct a laser beam **30** operable to perform the actions required to be done on the biological tissue **12** during a surgical procedure and sense the results. These actions include surgical ablation (removal of matter).

The robot arm **105** comprises a manipulator **23** and a robot arm controller **106** which is specifically designed to operate and control the manipulator **23.** In the embodiment, the robot arm **105** advantageously allows the end effector **28** to be optimally positioned and held stable relative to the biological tissue **12** by the system **10** for the at least one action of work to be performed correctly. The robot arm **105** is securely mounted to a base unit **27** providing a stable platform sufficient for the range of motion required during the operation of the system **10.** The robot arm **105** has at least 5 degrees of freedom and contains safety features that allow for it to be used in close proximity to people by sensing resistance to movement. The robot arm controller **106** connects to the controller **100** which is operable to guide and monitor its activity.

Controller **100** is operable, under control of the electronic program instructions from storage **18,** to receive input via the input means **22,** combining and interpreting input comprising safety, localisation, surgical planning and sensor data in real time, and to utilize and control sensing for precision control, safety and localization, representations of the biological tissue **12** and work to be done, via machine perception and other means, process the input and, on the basis of the processing, control the positioning means **25** and the tool **24** to work the biological tissue **12** under the supervision of the surgeon **16.** The biological tissue **12** is worked by the tool **24** performing the at least one action of work thereon. In the embodiment, controller **100** comprises a computer system that communicates and controls the components of the system.

In the embodiment, the components of the system **10** required for the functioning of the tool **24** predominantly reside in a housing comprising a base unit **27.** The aforementioned components, comprising laser related components, particle filtration related components, water spray components, controller **100,** sensor controllers **103,** dynamic focusing optics controller **137,** storage **18,** hardware failsafe watch dog **175,** control systems, and power related components of the system **10,** will be discussed in further detail hereafter.

System **10** uses a positioning sensor **170,** which may also be referred to as a computer assisted surgery (CAS) navigation sensor, to track in three-dimensions the location and movement of the biological tissue **12** being worked on. The positioning sensor **170** is operatively positioned high up at an elevated location, either attached to a roof, wall, arm, or stand, with a clear field of view to the biological tissue **12** and surrounds. Several existing systems are commercially available and used in current surgical techniques. The typical process for a computer navigated TKR is to insert pins into the femur and tibia bones and attach a fiducial marker **70** - reflective arrays of markers that can be recognised and oriented by a positioning sensor **170.** The fiducial marker **70** position data is streamed to the controller **100** by the positioning controller **171** to assist in correctly positioning and aligning the resections. This input data is the primary method of the system **10** for understanding the location and movement intraoperatively of the biological tissue **12** being worked on. Fiducial markers **70** are also attached to surgical tools used in the procedure as the current gold standard method to position the resections in the correct place with the right alignment.

System **10** can also use the laser to indelibly mark one or more visually trackable patterns on the biological tissue creating a lasered (i.e. formed by the laser e.g. laser inscribed) fiducial mark **71.** A sufficiently high speed (for example operating at greater than about 60 Hz) optical imaging sensor can provide high-resolution imaging that the system **10** can use to detect movement of a mark by comparison against prior images, especially laterally from the point of view of the tool where the most risk lies for errors in targeting the biological tissue **12** for ablation. A lasered fiducial mark **71** can supplement the tracking provided by the aforementioned fiducial marker **70.**

The input to system **10** is appropriate to biological tissue **12** to be worked and the work to be performed. In the embodiment, the input comprises details of and/or associated with one or more of biological tissue **12,** patient **14,** the orthopaedic surgical procedure, one or more components of system **10,** and the surrounding environment. The details comprise pertinent data and/or information.

The data and/or information may be obtained by one more of retrieving, receiving, extracting, sensing, and identifying it, from one or more sources. The one or more sources of data and/or information may be part of system **10,** reside on a component of system **10** such as storage **18,** and/or be or reside elsewhere, remote from system **10.**

The data and/or information comprises: experimental and historic data; preoperative data, including pertinent medical imaging of biological tissue **12** and the patient **14** and clinical/surgical plans for the patient **14;** and data obtained via intraoperative sensing during the procedure (discussed further below). The data and/or information may comprise joint kinematics data generated via gait analysis using at least one inertial measurement unit (IMU). An IMU is an electronic device operable to measure and report on a body's specific force, angular rate, and sometimes the magnetic field surrounding the body, using a combination of accelerometers and gyroscopes, sometimes also magnetometers. In embodiments of the invention, data and/or information may also be generated by and/or obtained from devices, including, for example, activity trackers (such as those provided under the trade mark FITBIT^{™}), smart braces, and smart splints.

In the embodiment of the invention, the display **20** for displaying the user interface **21** and the user input means **22** are integrated in a touchscreen. In alternative embodiments, these components may be provided as discrete elements or items.

A touchscreen display **20** is operable to sense or detect the presence and location of a touch within a display area of the system **10.** Input commands to system **10** in the form of sensed "touchings" of the touchscreen display **20** are inputted to the system **10** as commands or instructions and communicated to the controller **100.** It should be appreciated that the user input means **22** is not limited to comprising a touchscreen, and in alternative embodiments of the invention any appropriate device, system or machine for receiving input, commands or instructions and providing for controlled interaction may be used, including, for example, a keypad or keyboard, a pointing device, or composite device, and systems comprising voice activation, voice and/or thought control, and/or holographic/projected imaging.

Typically, the user is a person suitably qualified and experienced to use the system **10** to perform the work on the biological tissue **12,** such as an orthopaedic surgeon **16** in the embodiment. The user is a trained medical professional who supervises and directs the system **10** via the touchscreen display **20** of the user interface **21.**

The display **20** and user interface **21** are positioned in a convenient location close to the surgeon **16** to maintain good visibility and easy interaction.

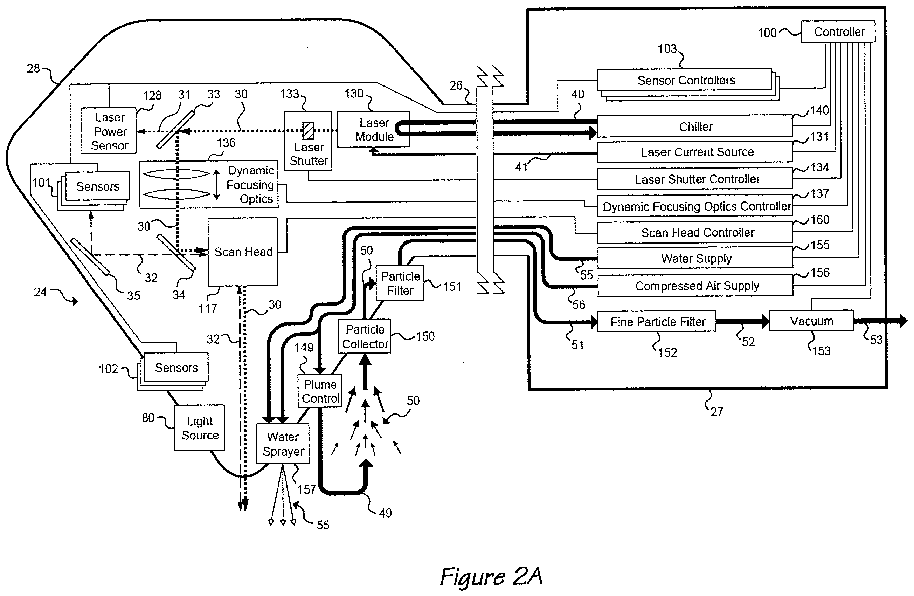

**Figure 2A** of the drawings depicts the preferred embodiment of the tool **24** as an end effector **28** comprising various components and connections to other components in the base unit **27** via the conduit **26.**

A laser module **130** is operable to emit a coherent light beam **30** at a predefined wavelength suitable for the ablation of biological tissue **12.**

A laser current source **131** is operable to supply the laser module **130** with electrical current required to generate the light beam **30.** The controller **100** is operable to select different parameters in the laser current source **131** to modify the resulting light beam **30** that is generated.

Cooling means in the form of a chiller **140** is operable to cool the laser module **130** via circulating coolant **40** to prevent it from overheating along with other components of the system **10,** including scan head **117** and water **55,** as discussed in further detail hereafter

A laser shutter **133** is operable to protect against the accidental emission of light from the laser module **130** using a switchable physical barrier that blocks the light beam **30.**

A laser shutter controller **134** is operable to control the functioning of the laser shutter 133 as directed by the controller **100.**

Dynamic focusing optics **136** are operable to dynamically change the focal length and focus beam diameter of the light beam **30** at the target distance determined as desirable by the controller **100** via the dynamic focusing optics controller **137.**

Mirror **33** is operable to reflect the light beam **30** at an alignment for the correct functioning of the dynamic focusing optics **136.** The mounted position of mirror **34** is adjustable to ensure the alignment that can be calibrated depending on the position of the laser module **130** and correct path into the dynamic focusing optics **136.** In the preferred embodiment, mirror **33** is partially reflective such that it is operable to beam split a very small predetermined ratio of transmitted beam **31** to a laser power sensor **128** that is operable to sense the power output of the laser module **130.** The controller **100** can calculate the total power output of the laser module **130** by compensating for the known ratio of sensed transmitted energy. In another embodiment where it is not necessary to dynamically sense the laser energy the mirror **33** can be fully reflective.

Mirror **34** is a dichroic filter that is operable to reflect light in the wavelength band of the light beam **30** into the beam entrance of a scan head **117** and transmit light outside that wavelength band along the light path **32.** The mounted position of mirror **34** is adjustable to allow the reflective alignment to be calibrated depending on the path of light beam **30** through the dynamic focusing optics **136** and the correct path into the scan head **117.**

The precision of a current state-of-the-art robot arm **105** is not sufficient to be the only method of directing the laser beam **30** from the end effector **28** to the biological tissue **12** for ablation. A means of fine motion is operable to allow for a secondary method of directional targeting of the laser beam **30** at micron-scale accuracy.

In the preferred embodiment, a scan head **117,** typically comprising two galvanometer scanner aligned mirrors (not shown), is used as the fine motion means to rapidly retarget the laser as directed by a scan head controller **160** operating as fine motion controller **111 of** **Figure 1****.** A scan head **117** typically has an f-theta lens that corrects the focus distance across a flat target plane, however as our target will be a freeform shape the embodiment does not need an f-theta lens and uses dynamic focusing optics **136** instead. Other embodiments with different fine motion means, such as rotating polygonal mirrors, tip/tilt mirrors, or Stewart platforms, will be discussed in further detail hereafter.

Mirror **35** is operable to reflect the light path **32** to the set of sensors **101** in direct line-of-sight with the path of laser beam **30.** The mounted position of mirror **35** is adjustable to allow the reflective alignment to be calibrated with the path of light beam **30** and the set of sensors **101.**

The set of sensors **101** comprise a plurality of components requiring sensing and/or emitting light along the same path as the laser beam **30** to the target of ablation on the biological tissue. The set of sensors **102** comprises a plurality of components positioned on the surface of the end effector **28** operable to sense and/or interact with the biological tissue or environment from its own vantage point. For intraoperative safety, localisation and precision shaping there is a requirement to noninvasively identify the biological tissue **12** prior to laser shaping. The sensors will be discussed in further detail hereafter.

A light source **80** is operable to provide illumination such that the set of sensors **101/102** can correctly and optimally sense the biological tissue **12** and environment. For various sensors, such as a hyperspectral imaging sensor **180** which will be discussed in further detail hereafter, this illumination will be operable across the range of wavelengths and modalities being sensed.

Lasers suitable for surgical ablation generate a light beam **30** at a wavelength that is strongly absorbed by water, a common and abundant component of the biological tissue **12.** Water has a strong absorption peak at a wavelength of about 3 µm. Example laser sources which operate in this particular wavelength range and are particularly suited to laser ablation procedures such as disclosed herein include Er:YAG solid state laser systems having an emission wavelength of about 2940 nm. The instantaneous heating of the water is the process by which material is ablated from the surface of the biological tissue **12.** If there is not enough water to efficiently absorb the energy of the laser light beam **30** then the ablating effect is reduced, and the biological tissue **12** is in danger of merely carbonising. If there is too much water too much energy is wasted merely flash boiling water and again the ablating effect is reduced, however there is little danger of carbonising the biological tissue **12.** Given these two extreme cases, there exists an optimal amount of water whereby the most biological tissue **12** is ablated without carbonising for the least amount of energy used in the light beam **30.**

Where there is too much water on the biological tissue **12** there is no direct way for the system **10** of the embodiment to reduce it other than expend additional energy with the light beam **30** vaporising it. An indirect way would be to rely on the surgical team to use suction near the target point to remove some water.

It is easier to introduce additional water to the biological tissue **12** than to remove it. In the preferred embodiment, this comprises a water supply **155** of a suitable sterile state provided via a consumable container or piped supply, and operable to provide water **55** for spraying onto the surface of the biological tissue **12** to be ablated.

In preferred embodiments, the sprayer liquid comprises a solution such as, for example, water or a water-derived solution or similar as would be appreciated by the skilled addressee. Suitable solutions would preferably have a strong absorption of light at the wavelength of the laser beam generated by laser module **130** so as to effect instantaneous heating of the sprayer liquid which assists the ablation process making it more efficient, whilst simultaneously acting as a coolant for the biological tissue **12** to protect the biological tissue **12** against carbonisation.

Directing means in the form of the water sprayer **157** is operable to irrigate the biological tissue **12.** This advantageously allows for more efficient ablation and assists in protecting the biological tissue **12** against carbonisation. In alternative embodiments of the invention, liquids other than, or additional to, water may be used.

Compressed air supply **156** is operable to propel the water **55** through the water sprayer **157.** In the preferred embodiment, this comprises a compressor taking sterile air from the surgical environment which is then compressed and the compressed air **56** is channelled to the water sprayer **157.** In alternative embodiments of the invention, containers or a piped supply may be used as the compressed air supply **156.**

The ablation of biological tissue **12** results in a plume **50** of vaporised and fragmented material ejected from the surface. The plume comprises particulates of submillimetre-scale and submicron-scale. Some microscopic particulates pose a health hazard to the operating staff about the system **10** and the patient **14,** as inhaling the particles can be damaging to the respiratory system. A preferred solution to this problem is for the system **10** to comprise extraction means to capture and filter the plume **50.**

In the preferred embodiment, the extraction means comprise a particle collector **150,** a particle filter **151,** a fine particle filter **152** and a vacuum **153** operable to capture the plume and extract the particulates via filtration.

A particle collector **150** is comprised of a plurality of openings operatively positioned close to where the ablation plume **50** is ejected to ensure that it collects the majority or all the particulates. The plume **50** is initially passed through a particle filter **151** that progressively filters smaller particles - given the volume of ablation material it is important not to fill a single high efficiency (fine) filter. The filtered air **51,** substantially reduced in volume of particulates, is channelled to a fine particle filter **152** that will filter and retain the remaining finer particulates not captured by the particle filter **151.** A vacuum **153** provides the pressure to draw the air into the filtration system and expel the cleaned air **53** from the fine particle filter is channelled to the environment.

In the preferred embodiment, the particle filter **151** retains the majority of the particulates in the plume **50** and is a consumable filter shield **43,** discussed in more detail herein, which can be replaced for each operation. The fine particle filter **152** comprises a high efficiency particulate air (HEPA) filter.

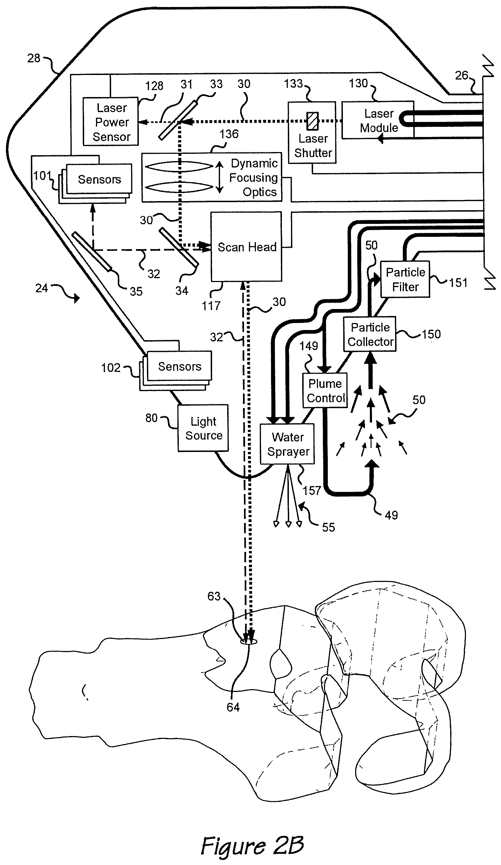

**Figure 2B** depicts an example usage configuration of the end effector **28** of the system **10** of **Figure 1** or **Figure 2A** for restructuring of bone tissue of a patient during a surgical procedure. Laser beam **30** from laser module **130** impacts the biological material (e.g. bone) **12** with a spot size **64** defining the interaction region **63.** Typical spot sizes for operation of the systems and methods disclosed herein range between about 300 and about 1000 µm to give the requisite laser beam fluence in the interaction region **63** necessary for efficient operation of the system **10** for laser ablation processes of biological tissue **12** such as, for example bone.

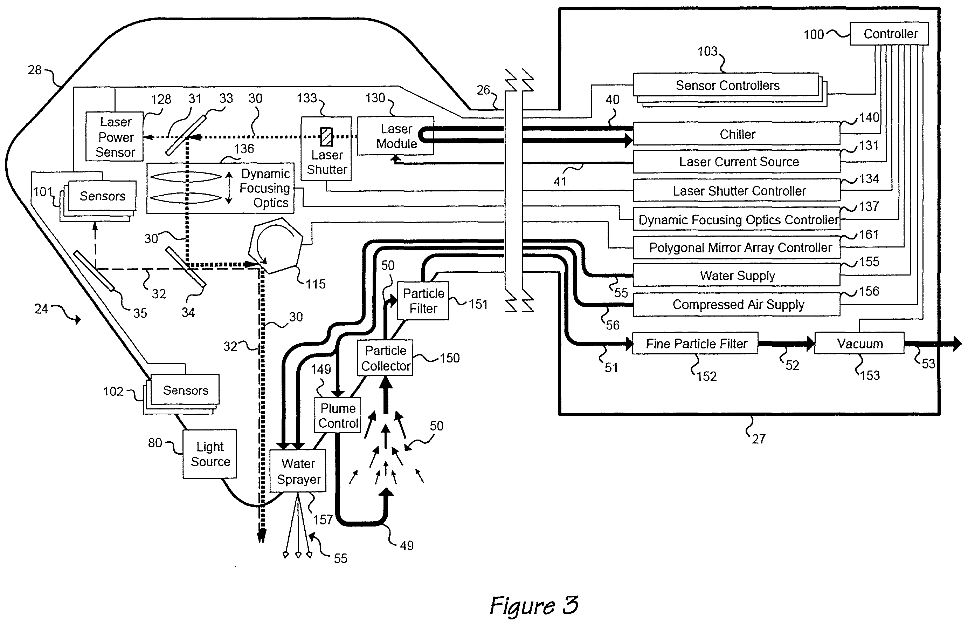

**Figure 3** of the drawings depicts an embodiment of the tool **24** as an end effector **28** of the system **10** utilising the alternative fine motion means of a rotating polygonal mirror array **115** as directed by a polygonal mirror array controller **161** operating as fine motion controller **111** of **Figure 1****.**

The polygonal mirror array **115** is a set of mirrors arranged at incrementing angles around a polygon that that is rotated at a precisely constant speed.

The angles of the mirrors mean that the light path **32** sweeps in several lines with a stride distance between them (looking somewhat similar to a square barcode with lines of equal width and distance between them). Eventually, upon a complete revolution of the array, the light path **32** returns to the start of the first line and the pattern repeats.

The controller **100** times the light beam **30** pulses to coincide when the polygonal mirror array **115** is targeting a surface location requiring ablation.

The advantage of this method of fine motion is that the rotating polygonal mirror array **115** is simple and requires less expensive components.

The disadvantages of this method is that the controller **100** needs to wait until the polygonal mirror array **115** rotates into the position for the required targeting and there is a set pattern of ablation, which may be inconvenient for ideal ablation in the minimum time required.

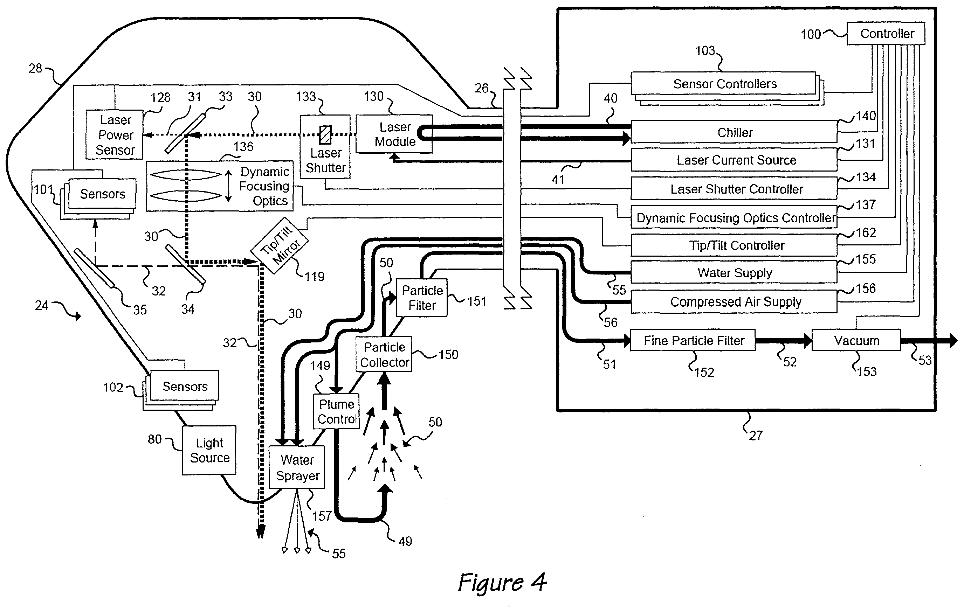

**Figure 4** of the drawings depicts an embodiment of the tool **24** as an end effector **28** of the system **10** utilising the alternative fine motion means of a tip/tilt mirror **119** as directed by a tip/tilt controller **162** operating as fine motion controller **111** of **Figure 1****.**

The tip/tilt mirror **119** is a small device with a piezo controlled mirror at an end thereof. The tip/tilt mirror **119** can be angled in the X/Y axis.

This is similar to the scan head **117** but potentially faster, with the downside of having a lower targeting angular range.

In an embodiment of the invention using tip/tilt mirror **119,** the tip/tilt mirror **119** is aligned at 45° to the light beam **30** such as that when the tip/tilt mirror **119** is in the 'neutral' position the light beam **30** is reflected directly down (at 90° to the incident light path). In this way the tip/tilt mirror **119** can redirect the laser beam **30** in a cone shaped targeting range.

A primary advantage of such a technique is near instantaneous and precise targeting of the light beam **30.** A fast moving tip/tilt mirror **119** means that the system **10** can reposition the laser beam **30** and pulse the laser faster causing a greater volume of biological tissue **12** to be ablated.

A disadvantage may be that the range of the targeting cone is less than that using the laser scan head **117.** It would be advantageous to place the tip/tilt mirror **119** as close to the top of the end effector **28** as possible to maximise the targeting angle, but that may also result in shielding that opens outwards, lessening the effect of protecting against objects getting in the way of the laser beam **30.**

Advantages arising from use of the tip/tilt mirror **119** include: small inertial mass (almost no influence of acceleration); fast movement of laser beam **30;** and ease of implementation.

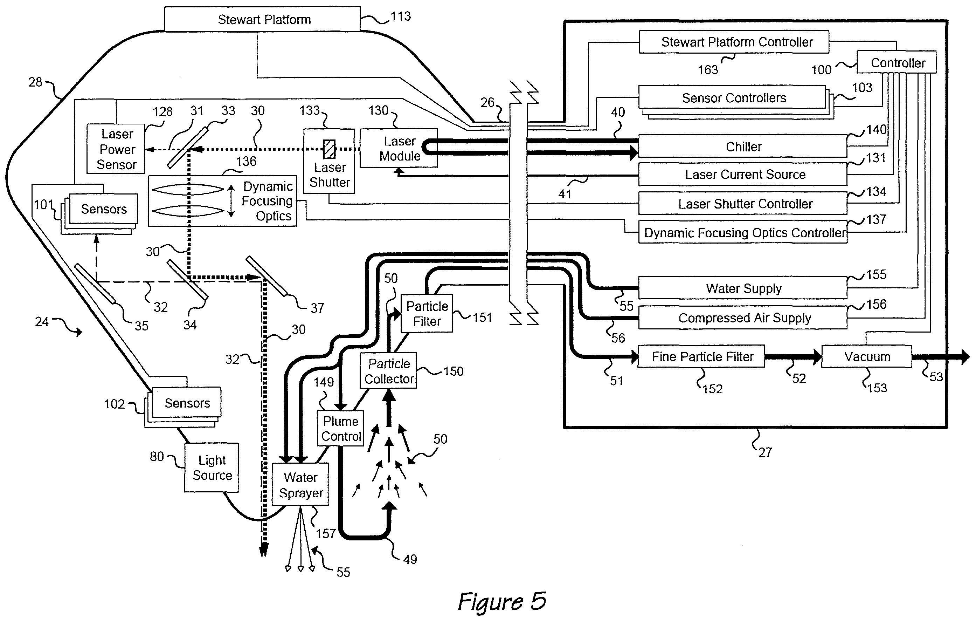

**Figure 5** of the drawings depicts an embodiment of the tool **24** as an end effector **28** of the system **10** utilising the alternative fine motion means of an actuator in the form of a Stewart platform **113,** which may also be referred to as a "hexapod", as directed by a Stewart platform controller **163** operating as fine motion controller **111 of** **Figure 1****.**

The Stewart platform **113** is a type of parallel robot that has six prismatic actuators, which may be, for example, hydraulic jacks or electric actuators, attached in pairs to three positions on a baseplate of the Stewart platform **113,** crossing over to three mounting points on a top plate of the Stewart platform **113.** Items, such as devices, placed on the top plate can be moved in the six degrees of freedom in which it is possible for a freely-suspended body to move. These are the three linear movements x, y, and z (lateral, longitudinal, and vertical), and the three rotations pitch, roll, and yaw.

Using a Stewart platform **113** is a solution that allows for very precise XY linear movement with the benefit of Z linear movement to maintain a set distance from the target surface contours if required. Depending on the solution it also allows for angling of the laser beam **30** as well as linear movement. Given enough weight bearing capacity it is also possible to allow for other components and covers to be also mounted directly.

Advantages provided by use of Stewart platform **113** include: a wide range of movement; 3D movement (maintaining focus); and all tools (water sprayer **157,** plume collector **150,** laser module **130,** sensors of the set of sensors **101** and **102)** maintain the same movement.

There may be disadvantages arising from the use of a Stewart platform **113** such as: inertial mass; acceleration and deceleration (complicated laser control); complicated implementation in the overall system (minimum **12** DOF); and obscuring of other sensors on the end effector **28.**

The principal disadvantage of inertial mass has a significant effect on the amount of time it takes to direct the laser beam **30** at the next target location on the biological tissue **12.** A scan head **117** or tip/tilt mirror **119** can individually target at least 400 laser pulses per second across any geometry, which a Stewart platform **113** cannot match.

The mass of the end effector means that even a moderate weight of several kilograms will likely cause issues with momentum. Moving the platform to micron-scale accuracy may cause it to "overshoot" as it may not be able to accurately stop the movement in time, or the opposite problem of backlash. Some of these problems can be mitigated by getting the end effector **28** up to speed prior to ablating to keep it going at a set speed and firing the laser beam **30** in a timed manner (as with a pendulum, for example).

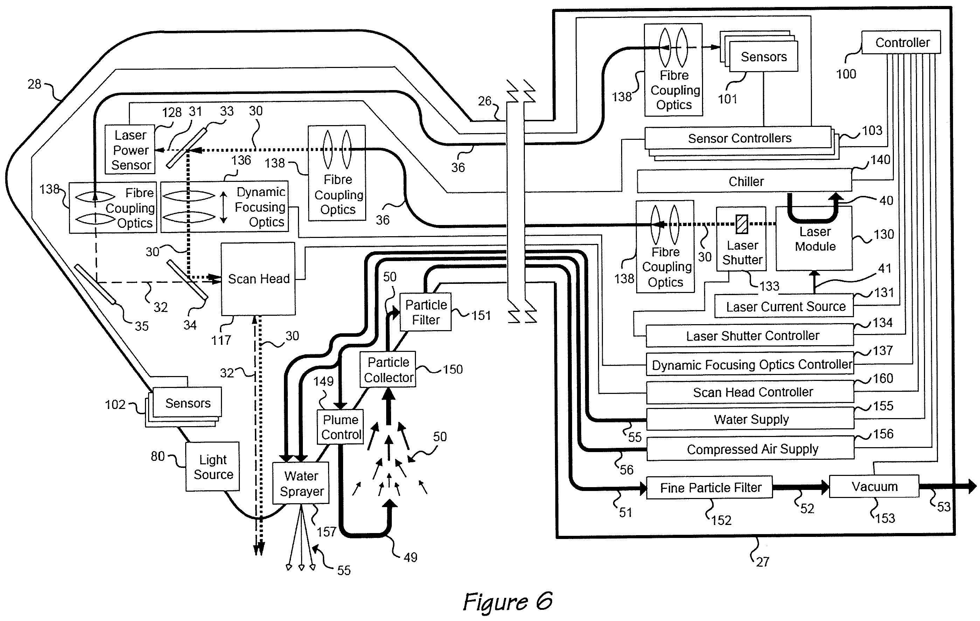

It should be appreciated that alternative laser power transmission to the end effector **28** is possible. In an alternative embodiment of the invention depicted in **Figure 6** of the drawings, rather than mounting the laser module **130** in the end effector **28,** it is possible to have the light beam **30** transmitted to the end effector **28** via a fibre optic 36 with fibre coupling optics **138** from the laser module **130** mounted within the base unit **27.** The use of fibre optic **36** is to be considered as merely the preferred embodiment of the standard methods of transmitting laser energy, including, but not limited to, a wave guide or articulated arm.

Similarly, in the alternative embodiment of the invention depicted in **Figure 6** of the drawings, the set of sensors **101** can be housed in the base unit **27** and the light path **32** is transmitted from the end effector **28** via a fibre optic **36.**

An advantage of this alternative method includes that less space is required, and weight is reduced, in the end effector **28.** Depending on the components that need to be incorporated into the end effector **28** it may be that the physical space isn't available or that the required alignment with the dynamic focusing optics **136** isn't practical. In such an embodiment, rather than wide cabling required for carrying power and water from the base unit **27,** all that is required is a single thin optical fibre **68.**

Such an advantage may be minimal in embodiments where other cabling is required, for suction and sterile air/water for example.

It should be kept in mind that there may be more complex coupling optics required to channel the laser light beam **30** into the fibre optic **36** and decouple the laser light beam **30** back out of the fibre optic 68 at the end effector **28.** There may also be a substantial laser power loss by coupling through the optical fibre. Generally speaking, fibre optic **68** itself may have a 25% power loss and the coupling/decoupling optics required may contribute a further 23% loss. These factors mean that a more powerful laser current source **131** is required which in turn has a greater cost and running electrical power requirement. Furthermore, generally speaking, an optical fibre is fragile and will break if bent too far or knocked with enough shock force.

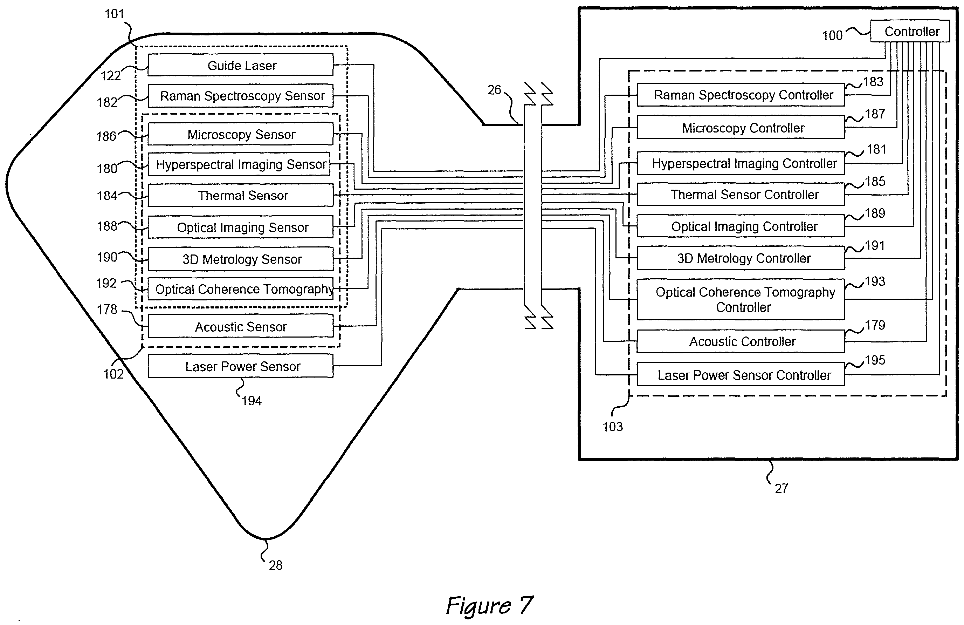

**Figure 7** of the drawings depicts an embodiment of the set of sensors **101** and the set of sensors **102** in the end effector **28** with connections to associated controllers in the set of sensor controllers **103** in the base unit **27** via the conduit 26. It should be noted that in some embodiments a sensor may be in the set of sensors **101** operatively positioned to sense the light in the line-of-sight light path **32** and/or sense the biological tissue from another vantage point as part of the set of sensors **102.**

Individual sensors, in the process of providing input to and being directed by the controller **100,** are operable to monitor, sense and gather or measure sensor data and/or information associated with or relating to one or more characteristics, properties and/or parameters of the system **10,** the biological tissue **12,** the patient **14,** the surrounding environment, components, systems or devices associated therewith or coupled thereto.

A guide laser **122** allows the system **10** to provide visual feedback to observers, such as the surgeon 16 and members of the surgical team, of proposed ablation target areas on the biological tissue **12.** This advantageously increases user confidence in the system **10** by clearly providing visual feedback on the biological tissue **12** rather than just through the display **20.** Without the guide laser **122** it may sometimes be unclear exactly where the system **10** is planning to ablate, or is currently ablating, as the ablation laser beam **30** is not visible to the naked eye in the embodiment. The guide laser **122** is operated by the controller **100.** The guide laser is a visible light laser, and in the preferred embodiment the guide laser is a red laser.

A Raman spectroscopy sensor **182** is operable to observe vibrational, rotational, and other low frequency modes in the biological tissue **12** as directed by the controller **100** via the Raman spectroscopy controller **183.**

Raman spectroscopy relies on inelastic scattering of monochromatic light, usually from a laser source, and is operable to provide additional information from a single point on the biological tissue **12.** The system **10** is operable to use Raman spectroscopy for material identification in surgical procedures. In addition to acting as a guide, the above-mentioned guide laser **122** beam can serve the secondary purpose of acting as a red laser light source for Raman spectroscopy. In a Raman scattering process a small fraction of a red laser beam's photons are inelastically scattered, that is they lose energy to the molecules that make up the target sample. The amount of energy lost per photon is related to the vibrational energy of the molecule. So, for example, bone contains hydroxyapatite (Ca5(PO4)3(OH)) and the PO bond has a characteristic Raman line at 959 cm-1. The presence of this line in the Raman scattered light indicates that the guide laser **122** is on bone. Depending on the work action to be performed and analysis and control decisions made (discussed in further detail below), if bone is the target and other conditions are satisfied, then the controller **100** is operable to activate the laser beam **30** and ablate the biological tissue **12.** Also, other joint tissues, such as cartilage and ligament, have characteristic Raman spectra that similarly allow them to be identified.

A microscopy sensor **186** is operable to provide a detailed magnified visual image of the biological tissue **12** as directed by the controller **100** via the microscopy controller **187.** The system **10** is operable to use information gathered by the microscopy sensor **186** to assist in the detection of biological tissue **12** composition via visual comparison to known samples. The detailed resolution imagery generated can also be provided to the surgical team to provide a better viewpoint of the target which may be impossible to see from a vertical orientation given the necessity of the positioning of the end effector **28.**

A hyperspectral imaging sensor **180** is operable to detect the intensity and frequency of reflected, or absorbed, light across a wide spectrum (beyond visual range) as directed by the controller **100** via the hyperspectral imaging controller **181.** By illuminating the target with a light source **80** that covers the spectral range of the hyperspectral imaging sensor **180** samples can be taken of the reflected light. In the preferred embodiment, the spectral range from 400nm to 1000nm is used.

The varying intensities contain a signature that is used by the system **10** to predict the type and composition of biological tissue **12.** This prediction can be carried out with machine learning, as will be described in further detail.

The hyperspectral data can be used with machine learning to identify the types, properties and composition of biological tissue **12.**

A thermal sensor **184** is operable to detect radiation in the mid to long-infrared range of the electromagnetic spectrum to map out the temperature of the biological tissue **12** as directed by the controller **100** via the thermal sensor controller 185. During and after laser shaping the system **10** is operable to noninvasively assess the thermal impact and the results can be used to optimise the laser parameter selection in subsequent ablation runs.

An optical imaging sensor **188** is operable to provide a visual image of the biological tissue **12.** The images can be analysed to assist in the prediction of the type and composition of biological tissue **12.** It can also be used at high speed to track objects in the field of view as directed by the controller **100** via the optical imaging controller 189. The aforementioned lasered fiducial mark **71** can also be tracked with an optical imaging sensor.

A 3D metrology sensor **190** is operable to detect the geometry of the surface of the biological tissue **12** as directed by the controller **100** via a 3D metrology controller 191. Information gathered by which is used by the system **10** to determine the geometry of a surface without contact. The geometry of the surface of the biological tissue **12** is very important for the system **10** to be able to determine the shape prior to and after ablation.

There are several different types of technology to sense a 3D geometry, including time-of-flight, laser triangulation, structured light projection and modulated light. In order to achieve the required micron-scale resolution, the system **10** uses structured light projection technology, line profile sensor or a point profile sensor, such as a chromatic confocal sensor. In an embodiment, a structured light scanner projects patterns of light on the subject and two offset cameras look at the deformation across the surface - a process that takes fractions of a second and can scan a relatively large area quickly. In the preferred embodiment, a chromatic confocal sensor is part of the set of sensors **101** and takes readings as the fine motion means traverses the light beam **30** across the surface, building up a complete geometry over time.

Optical coherence tomography (OCT) sensor **192** is operable to capture micron-scale resolution, three-dimensional images of the biological tissue **12** as directed by the controller **100** via an optical coherence tomography controller **193.** OCT uses light to capture micrometre-resolution, three-dimensional images based on low-coherence interferometry. This allows it to penetrate scattering media such as biological tissue up to about 1000 to 2000 µm below the surface of the biological tissue **12** to obtain data of the underlying structure of the tissue to be ablated. The system uses data obtained from OCT sensor **192** as a mechanism to detect the surface and underlying tissue composition for planning the ablation process for maximum ablation efficiency with minimal thermal affect to the surrounding tissue capable of causing thermal-induced damage to the surrounding tissue.

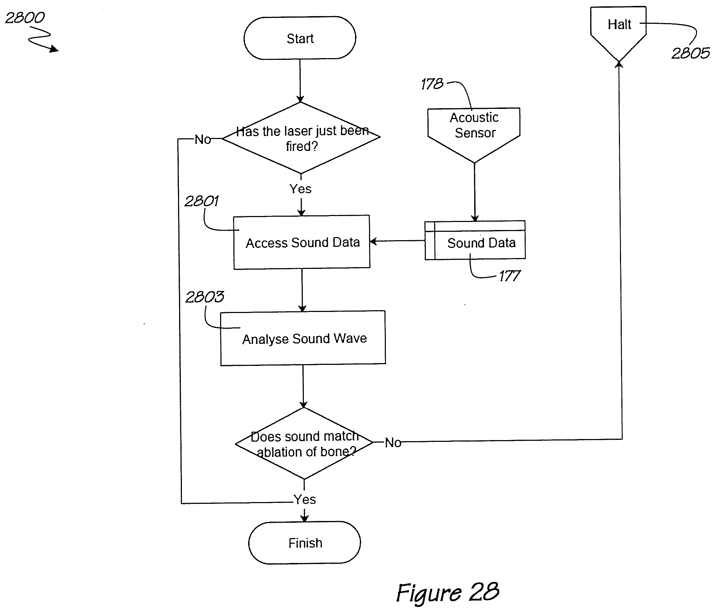

While an ablation is taking place the system **10** will not be able to sense the environment in the same microsecond timeframe and there is a risk that the material targeted is different than expected, or a foreign object is introduced into the light beam **30.** One possible way to mitigate this risk, and utilized in the embodiment, is the use of an acoustic sensor 178 which is operable to measure the pressure waves from the environment and around the biological tissue **12.** With reference to method **2800 of** **Figure 28****,** the distinct pressure waves that a laser light beam **30** makes ablating material, such as biological tissue **12,** can be measured and, via analysis of the data measured, the system **10** is operable via the acoustic controller **179** to determine in real-time if an unexpected material has been hit. Hard biological tissue sounds different to soft biological tissue, and any other types of material. By examining (preferably constantly) the sound of the previous pulse's ablation it is possible to halt operation of the system **10** if any sound is encountered that disagrees with the expected sound of the material type that was the target of the ablation. This safety feature may not prevent the first laser pulse from hitting an unexpected material, but it can prevent subsequent laser pulses that would have otherwise occurred prior to any overall re-sensing.

The sensing techniques above are used in the implementation of the invention to identify and monitor the biological tissue **12** and operate in conjunction with the shaping laser to deliver a safe, highly accurate and precise system **10** under intelligent control.

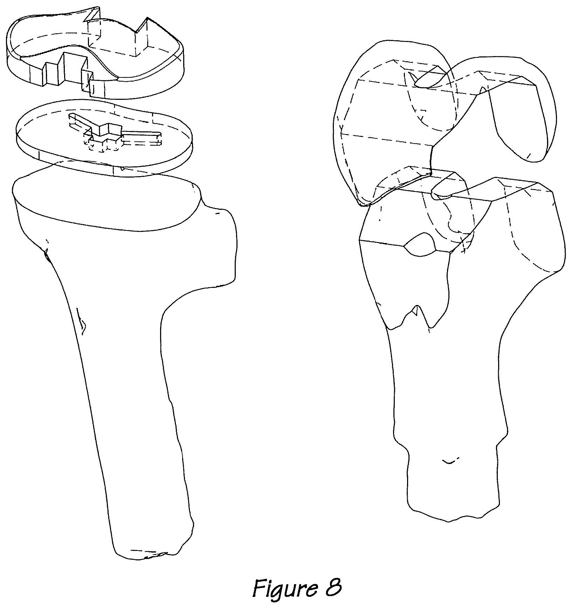

As will be described in further detail, the system **10** is operable to perform subtractive laser ablation that takes into account one or more relevant factors including thermal effect (that is to say, biological quality of residual tissue - non localized), biological make up of bone being ablated, flatness of residual surface, planar alignment and relationship to mechanical axis (global alignment). The laser light beam **30** may also ablate defined channels or holes, or other shapes or shaping as may be appropriate, into the bone that will allow for/accommodate an implant/prosthesis or one or more portions or components **13** thereof, as depicted in **Figure 8** of the drawings showing the matching resections **60** of the joint ends of a tibia bone and a femur bone.

In addition to bone, the laser light beam **30** is also operable to cut all human tissue, including skin, muscle, fat, meniscus, tendons, connective tissue, articular cartilage, capsule, ligament, and fascia of the patient **14.**

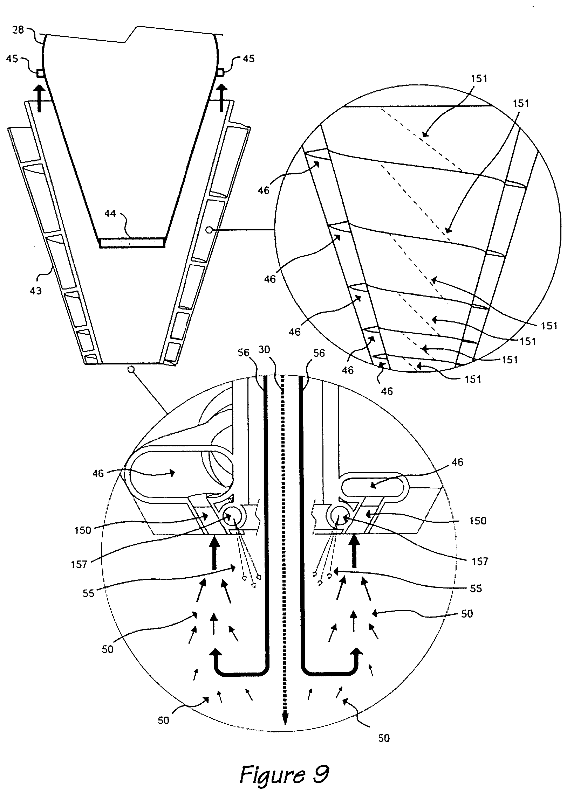

The consumable filter shield **43** will now be described, with reference to **Figure 9** of the drawings.

In the embodiment, an operational distance of the end effector **28** from the biological tissue **12** is between 150 and 300 mm. This distance is the collective working range for the sensors and light beam fine motion means. It also allows for a physical gap where an ablation plume can be ejected without immediately coating the outer surface of the end effector **28** that would result in contamination.

However, while the distance is helpful in preventing contamination it poses an increased risk of providing room for objects, and - more critically - portions of humans (other than the patient **14),** to accidentally be positioned between the end effector **28** and the biological tissue **12.** A primary danger is the laser beam **30** which could cause damage to soft biological tissue (of a human other than the patient **14)** that happened to be occluding the target when it fires. Although the risk of injury decreases exponentially the further the distance from the focus point (since the energy density or fluence in joules (i.e. radiant laser energy per cm²) decreases away from the focus) of the laser beam **30**, it is still a risk to members of the surgical team during a procedure. Another risk is a non-human object that is shiny or mirror-like causing a specular reflection of laser energy. This, however, is a very small risk as any reflected laser energy will be likely scattered and un-focused, with the additional protection of safety glasses being mandatory for the operating staff for eye protection in the embodiment during use.

In order to form a physical barrier to the danger zone defined by the vertical space between the biological tissue **12** and the end effector **28,** the system **10** comprises shielding for providing protection, having the form of an open-ended cone shaped consumable filter shield **43** in the embodiment. A larger open end of the consumable filter shield **43** is attached to the end effector **28** via attachment points **45** that securely hold the consumable filter shield **43** in place. A smaller open end of the consumable filter shield **43** provides a space, or gap, between itself and the target biological tissue **12.** Such a gap advantageously: allows for easy visual inspection by a user of the system **10,** such as a surgeon **16,** while an ablation action is occurring; serves as a precaution against collisions between a biological tissue **12** that is shifting in location and the consumable filter shield **43;** and allows working space, for example for retractors, suction, irrigation or other surgical tools.

Water **55** (or a suitable alternative) is applied to the biological tissue **12** via a plurality of water sprayers **157** that is operable to enhance the ablation process and act as a coolant for the biological tissue **12.**

As mentioned above, microscopic particles of the ablation plume **50** are a health hazard to the operating staff of the system **10** and the patient **14,** as inhaling the particles is damaging to the lungs. A preferred solution to this problem is for the consumable filter shield **43** to comprise particle collectors **150** in the form of a plurality of openings operatively positioned relatively close to the location where the ablation vapour is ejected to ensure that it collects the majority of the particulate material in the plume **50.** The vacuum **153** draws the plume **50** into the particle collectors **150** and into the spiral conduit **46** that encircles the consumable filter shield **43.**

The consumable filter shield **43** is operable to channel compressed air **56** out the smaller open end as a means of plume control **149.** The compressed air **56** flow directs the plume away from the light beam **30** and towards the particle collectors **150.**

An initial method of filtering and collecting the ablated material is through a series of "traps" and filters **151** built into a spiral conduit **46** around the consumable filter shield **43.** So, rather than the consumable filter shield **43** being a single layer of suitable material, such as plastic, it has an outer layer and an inner layer. In such an embodiment, a sloped divider, as illustrated in **Figure 9** of the drawings, can then have a series of angled filters 151 that will allow ablated material to be progressively filtered (largest to smallest particles) and collect in areas away from the direct suction airflow. A particle collector **150** is preferably fairly narrow in its cross-section. This will cause the air flow rate to be high (i.e. strong vacuum). The spiral conduit **46** can increase in height as it travels up the consumable filter shield **43.** This will in turn decrease the air flow as the cross-section becomes larger and heavier ablated material will lose velocity and fall to the bottom of the spiral conduit **46.** By having angled filters forming the traps **151** positioned along the spiral conduit **46** the ablated material will collect and not slide back down. By controlling the rate of increase in the spiral conduit **46** cross-section, the volume of the ablated material collecting at each point can be controlled from studying the relative percentage distribution of ablated material density. Preferably, by the time the spiral conduit **46** reaches the cavity where the vacuum tube connects, the spiral conduit **46** cross-section increases significantly and even fine ablated material can be collected.

By collecting a portion of the ablated material in the consumable filter shield **43,** to be disposed of after each surgery involving the system **10** in the embodiment, the total ablated material that needs to be collected by the fine particle filter **152** is reduced. This advantageously extends the working lifetime of the more expensive fine particle filter **152** which would otherwise become clogged with ablated material in a very short time given the approximately **50** cm³ of biological tissue **12** that is ablated in a TKR.

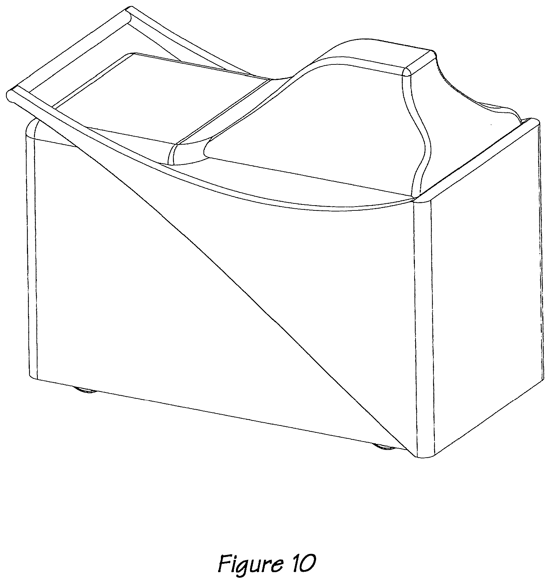

**Figure 10** of the drawings depicts an embodiment of the base unit **27**, containing all the equipment and consumables that makes up the system **10** and isn't installed in the end effector **28.** Some of the equipment is bulky and heavy, which makes it ideal to position low within the base unit **27** to lower the centre of gravity and stabilise the system **10** for operation.

Communication and conduit with the end effector **28** is via a bundled cable that is secured to, or within, the robot arm **105.** Amongst other things, the bundled cable contains electrical power **41;** coolant **40** (to/from); partially filtered air **51;** water **55;** compressed air **56;** control wires and/or fibre optics **36.** By partitioning the bundle into insulated conduits, coolant **40** from the chiller **140** can be packed closely with the water **55** tube along the length of the bundled cable. This advantageously results in the water **55** being cooled by virtue of the arrangement within the bundled cable alone, reducing the complexity of the system **10** and eliminating the need for a separate process of cooling the water **55.**

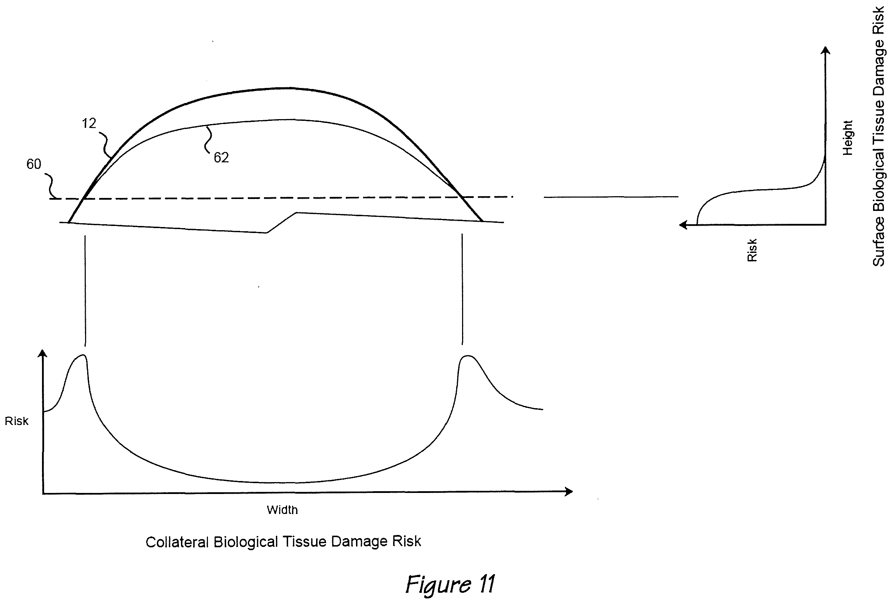

**Figure 11** of the drawings depicts a cross-section of biological tissue **12** with a planned resection **60** marked as a dashed line. The biological tissue **12** above the line indicates the amount of material requiring ablation and the material below the line should be left undamaged.

Each laser pulse ablates an extremely small volume of material. Given that each complete resection **60** requires a very large volume to be ablated it follows that there will need to be a significantly large number of laser pulses.

For a resection **60** to be completed within the required period of time the system **10** of the embodiment preferably performs these laser pulses almost as quickly as the generated subtractive laser beam **30** and means of fine motion will allow.

However, a competing requirement is that the ablation be safely and accurately performed. The predominant way for the system **10** to sense the biological tissue **12** and surrounding working environment is with the imaging or scanning sensors of the sets of sensors **101** and **102.** This provides the system **10** with a rich set of data to analyse and determine with confidence the points where the laser pulses are directed to ablate the correct material and that there is no other material in the way. However, some of the imaging and scanning sensors of the sets of sensors **101** and **102** take tens to hundreds of milliseconds to sense their environment. Were the system **10** to use these senses between each laser pulse it would take an unacceptable amount of time to complete a resection **60.** It is accordingly undesirable to re-sense the biological tissue **12** between each single pulse of the laser in the embodiment.

The solution of the embodiment is to batch the laser pulses into an ablation run **62** comprising a pre-calculated set of pulses at different locations across the surface of the biological tissue **12.** The system **10** is operable to perform each ablation run **62** after sensing the environment. The resection is performed in multiple ablation runs **62** that progressively reduce the tissue surface until the desired final resection 60 surface is achieved. Such operation may provide an optimal balance of sensing (assured) and speed.

As depicted in **Figure 11****,** the risk of collateral tissue damage is greatest around the edges of the volume of biological tissue **12** being ablated, representing the danger of miss-targeting with the light beam **30.** In addition, the risk of surface tissue damage is greatest approaching the final resection **60** location, representing the danger of continuing to ablate the surface beyond the ideal point.

Laser parameters of the system **10** will now be described.

The system **10** interacts with the surgical environment through several actuators including the laser module **130,** fine motion means (in the preferred embodiment a laser scan head **117),** robot arm **105,** plume control **149** and water sprayer **157.** Each of these components has several control parameters. These include:

| **Actuator** | **Parameter** | **Reasonable Range** | **Typical Range** |
|---|---|---|---|
| Laser | Average Output Power (W) | 0.1 - 2000 W | 0.5 - 200 W |
| Laser | Pulse Repetition Rate (Hz) | 1 - 2000 Hz | 200 - 1000 Hz |
| Laser | Pulse Duration (µs) | 0.1 - 1000 µs | 100 - 400 µs |
| Laser | Spot Size (µm) | 100 - 10000 µm | 300 - 1000 µm |
| Laser | Energy Density (J/cm²) | 0.1 - 1000 J/cm² | 10 - 150 J/cm² |
| Laser scan head | Traverse Speed (mm/s) | 1 - 40000 mm/s | 400 - 900 mm/s |

The range listed next to each parameter in the table above is an estimate of the upper bound, lower bound, and typical range for each parameter in the embodiment that is expected to perform a reasonable resection.

The laser parameters include: Average Output Power (in Watts) - the laser power of the light beam **30** generated; Pulse Duration (in microseconds) - the amount of time each pulse of laser is generated; Spot Size (in micrometres) - the diameter of the focused light beam **30;** Energy Density (in joules per square centimetre) - the laser energy delivered per unit area; and Pulse Frequency (cycles per second) - the number of pulses generated each second.

For flat plane ablation with a circular beam profile, in order to result in a smooth surface, the laser ablation must overlap in a pattern or grid such that the surface is reduced evenly.

Indicative research by the inventors suggests that a beam diameter of 200 to 600 microns and an overlapping pattern is sufficient to produce a flat ablated surface.

This means that any given point on the surface will be ablated multiple times due to the overlapping alone, even before any consideration of the depth of the ablation required. This accentuates the need for a quick method of firing the laser light beam **30** at the surface.

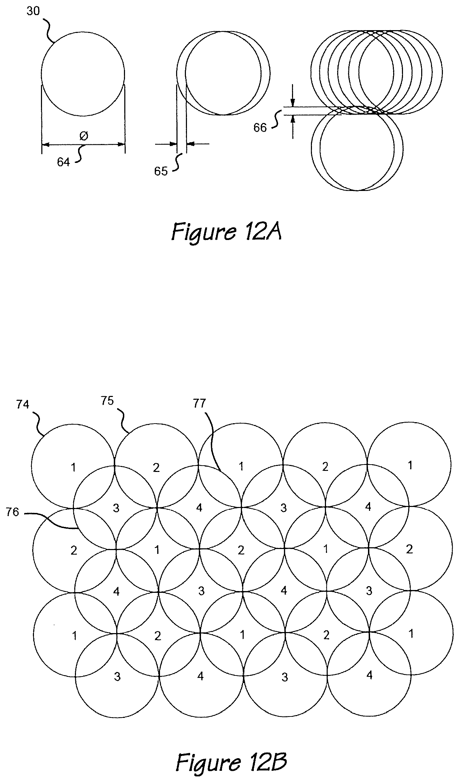

**Figure 12A** of the drawings depicts an example of preferred laser beam ablation channel dimensions. When creating a channel the light beam diameter or spot size **64** indicates the width of the channel with the subsequent pulse overlapping the first with a gap **65.** By continuing in this manner an ablated channel is created in the material. To create a final flat surface, multiple channels can be created with a stride **66** between them to allow for the sides of the channels to be removed.

However, multiple laser pulses across the surface in such a manner accumulates thermal impact, which may cause damage to the biological tissue **12** that is unwanted especially in the final resection **60** surface.

An alternative ablation pattern is an interleaved grid of laser pulses across the surface, as depicted in **Figure 12B****.**

The first series of pulses **74,** marked with a '1', are spaced to prevent a buildup of thermal impact in the same localized area. The second series of pulses **75,** marked with a '2', are spaced between the first series. The third and fourth series of pulses **76** and **77,** marked '3' and '4' respectively, overlap and complete the surface between the first and second series. In this manner an optimal coverage is achieved with the minimal thermal impact.

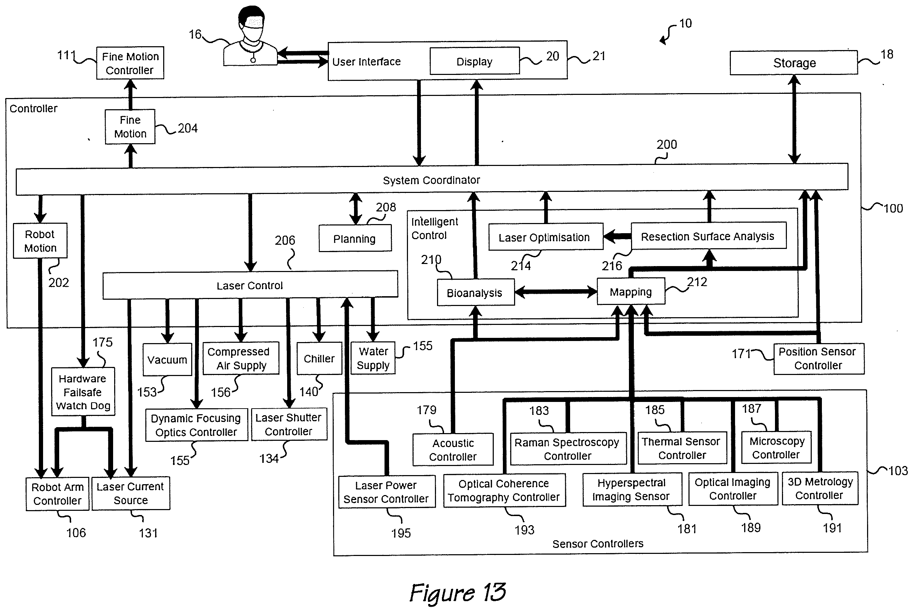

**Figure 13** of the drawings depicts architecture of the controller.

The controller **100** comprises system management software running on a computer. The controller **100** further comprises the software that manages the system **10.** The controller **100** optionally further comprises an artificially intelligent control system operable to fuse and interpret input received in real-time to operate the system **10.**

The responsibilities of the controller **100** include the following areas: communicating with the hardware components of the system **10;** processing the various information to decide on the actions to take given the circumstances; communicating the current operating status of the system **10** on the user interface **21;** and loading and storing data to and from storage **18.**

Particularly, the controller **100** is operable to utilise and control: sensors of the sets of sensors **101** and **102,** one or more of a plurality of representations pertaining to the biological tissue **12** and work to be done, machine perception, the robot arm **105,** and the laser related components. In such manner, the controller **100** is operable to implement actions in accordance with decisions made and commands received.

The controller **100** comprises processing means in the form of a processor.

The storage **18** comprises read only memory (ROM) and random access memory (RAM). Amongst other things, the storage 18 provides patient specific data and procedure plans for the system **10** to perform.

The system **10** is capable of receiving instructions that may be held in the ROM or RAM and may be executed by the processor. The processor is operable to perform actions under control of electronic program instructions, as will be described in further detail below, including processing/executing instructions and managing the flow of data and information through the system **10.**

In the embodiment, electronic program instructions for the system **10** are provided via software stored on the storage **18.**

As will be described in further detail, via the software, the system **10** is operable to perform functions including: extracting, converting and combining data and recording all real time data passing through the system **10.**

All data and information collected is distributed within the system **10** for use as described herein.

The system **10** also includes an operating system which is capable of issuing commands and is arranged to interact with the software to cause the system to carry out the respective steps, functions and/or procedures in accordance with the embodiment of the invention described herein. The operating system is appropriate for the system **10.**

The system **10** is operable to communicate via one or more communications link(s), which may variously connect to one or more remote devices such as servers, personal computers, terminals, wireless or handheld computing devices, landline communication devices, or mobile communication devices such as a mobile (cell) telephone. At least one of a plurality of communications link(s) may be connected to an external computing network through a telecommunications network.

The software, and other electronic instructions or programs for the computing components of the system **10** can be written in any suitable language, as are well known to persons skilled in the art. In embodiments of the invention, the electronic program instructions may be provided as stand-alone application(s), as a set or plurality of applications, via a network, or added as middleware, depending on the requirements of the implementation or embodiment.

In alternative embodiments of the invention, the software may comprise one or more modules, and may be implemented in hardware. In such a case, for example, the modules may be implemented with any one or a combination of the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA) and the like.

The computing means can be a system of any suitable type, including: a programmable logic controller (PLC); digital signal processor (DSP); microcontroller; personal, notebook or tablet computer, or dedicated servers or networked servers.

The processor can be any one or a combination of one or more custom made or commercially available processor, a central processing unit (CPU),a graphics processing unit (GPU), a data signal processor (DSP) or an auxiliary processor among several processors associated with the computing means. In embodiments of the invention, the processing means may be a semiconductor based microprocessor (in the form of a microchip) or a macro processor, for example.

In embodiments of the invention, the storage can include any one or combination of volatile memory elements (e.g., random access memory (RAM) such as dynamic random access memory (DRAM), static random access memory (SRAM)) and non-volatile memory elements (e.g., read only memory (ROM), erasable programmable read only memory (EPROM), electronically erasable programmable read only memory (EEPROM), programmable read only memory (PROM), tape, compact disc read only memory (CD-ROM), etc.). The respective storage may incorporate electronic, magnetic, optical and/or other types of storage media. Furthermore, the storage can have a distributed architecture, where various components are situated remotely from one another, but can be accessed by the processing means. For example, the ROM may store various instructions, programs, software, or applications to be executed by the processing means to control the operation of the system **10** and the RAM may temporarily store variables or results of the operations.

The use and operation of computers using software applications is well-known to persons skilled in the art and need not be described in any further detail herein except as is relevant to the present invention.

Furthermore, any suitable communication protocol can be used to facilitate connection and communication between any subsystems or components of the system **10,** and other devices or systems, including wired and wireless, as are well known to persons skilled in the art and need not be described in any further detail herein except as is relevant to the present invention.

Where the words "store", "hold" and "save" or similar words are used in the context of the present invention, they are to be understood as including reference to the retaining or holding of data or information both permanently and/or temporarily in the storage means, device or medium for later retrieval, and momentarily or instantaneously, for example as part of a processing operation being performed.

Additionally, where the terms "system", "device", and "machine" are used in the context of the present invention, they are to be understood as including reference to any group of functionally related or interacting, interrelated, interdependent or associated components or elements that may be located in proximity to, separate from, integrated with, or discrete from, each other.

Furthermore, in embodiments of the invention, the word "determining" is understood to include receiving or accessing the relevant data or information.

A system coordinator **200** encompasses the whole controller **100** and forms the 'backbone' that organises and interconnects all of the software components of the system **10.**

The responsibilities of the system coordinator **200** include, and it is operable to perform, the following: configure the components of the system **10;** manage the lifetime of the components; provide runtime services to the components; and enforce the overall safety of the system.

Components of the system **10** facilitating operations at the direction of the system coordinator **200** include: a fine motion component **204;** a robot motion component **202;** a laser control component **206;** a planning component **208;** a bioanalysis component **210;** a laser optimization component **214;** a mapping component **212;** and a resection surface analysis component **216.**

In further detail, the system coordinator **200** is operable to facilitate the following functions.

Configure: Operation of the system **10** is determined by the specific set of components that are included and configured by the system coordinator **200.**

Component versions: Each component of the system **10** has a specified interface that is used by the system coordinator **200** to access its functionality. As the system coordinator **200** controls the configuration and initialisation of the components, it is possible for any component to be replaced by a specially crafted alternative that honours the component's interface but performs a different function. This is useful for activities including development, testing, verification, quality control, and simulation.

Development: In the early stages of development of the system **10** there may be a version of most components that will perform no real function at all, but purely exist to satisfy the interface requirements with a non-functional 'stub'. These 'stub' components allow the system coordinator **200** to facilitate focused development on a specific single live component while the remaining system **10** is functionally neutral.

Simulation: Simulation versions of components can be created that will 'play back' pre-recorded or fabricated functionality and data. The primary purpose of this version of component is supporting integration testing and verification of functionality under success and failure scenarios. The secondary purpose of this version can be the recreation and analysis of the prior recorded live run.

Live: There is a single live version of each component that is used in the production build of the embodiment. This provides the real-world functionality required by the deployed product.

Manage: The system coordinator **200** is operable to manage the operation and lifetime of each component.

Component isolation: The run-time environment of each component can be set up and controlled by the system coordinator **200** depending on the purpose and circumstances of the instantiation. For development it may be that each component is executed in serial to assist in debugging and simplify the data flow for deterministic timing. For testing, validation, and live environments however, it is possible to set up separate processing threads for each component to allow for time critical functionality to be more finely controlled.

State management: The system coordinator **200** is operable to maintain the state of the system **10** as it transitions from task to task. This will invoke various functionality in each component as necessary for the current operation of the task at hand.

Component coordination/data flow: In the embodiment, data is not sent directly from component to component but rather through the system coordinator **200.** This allows for low coupling between the components for easier development and testing. It also provides an insertion point where the system coordinator **200** can perform additional functions on the data whilst in transit such as sanity checks, logging and data recording.

Runtime services: In the embodiment, each component requires access to global functionality that is common and synchronised across the system **10.** This may also encapsulate platform dependent functionality of the underlying services. This set of functionality is provided by the system coordinator **200** to each component.

Logging: Centralised logging allows each component to provide runtime status information that can be directed to several different destinations such as console, log file or database. This logging can be configured by the system coordinator **200** to levels of severity, verbosity and granularity.

Data recording: For any particular run of the system **10,** the data flow between the components can be recorded to a database for later analysis, verification or playback. This data recording can be used to configure a simulation component in later runs.

Timing (high resolution clock): A number of the actions of the system **10** require accurate timing. A high resolution clock is provided that is operable to allow for at least microsecond level timekeeping. Where the system **10** is in simulation mode this clock can be synchronised to an external source such as a provided data recording.

Safety: The safety of the system **10** is paramount, and the system coordinator **200** is ultimately responsible for the enforcement of fail-safe procedures in the embodiment. When a safety issue is detected, the system coordinator **200** is operable to ensure that each component is entered into an orderly fail-safe and the system **10** placed into a safe posture for further instructions by the operating staff. There are several ways that the system coordinator **200** can detect issues that would cause the system **10** to go into fail-safe, as will be described in further detail.

Component active fault: In the embodiment, each component can actively report a fault to the system coordinator **200** that will initiate the system fail-safe. This could be due to one or more of detected hardware failure, operations outside of safe parameters or unexpected or unknown operational situations, for example

Component passive fault: In the embodiment, each component constantly reports on its own correct functioning and operation. Should a component not report in a timely manner within the required specification the system coordinator **200** is operable to assume that it is in an undesirable bad or unknown state and initiate the fail-safe.

The system coordinator **200** itself is not assumed to be faultless and in the embodiment must report its own correct functioning to an independent hardware device, herein referred to as a watch dog **175** that will reset a hardware timer upon receipt of each successful report. Should the system coordinator **200** not report within the required time period the watch dog **175** is operable to trigger the hardwired shutdown of all hardware of the system **10.** For the robot arm **105** this may comprise a safety circuit and for the laser subsystem this may comprise activating a shutter and disabling the relevant power relay.

Regarding the watch dog **175,** the system **10** of the embodiment cannot rely solely on the correct operation of the controller **100** software, underlying operating system, drivers, or hardware. There is always the possibility of a failure of any one of these layers that may or may not be detectable by the software that is running.

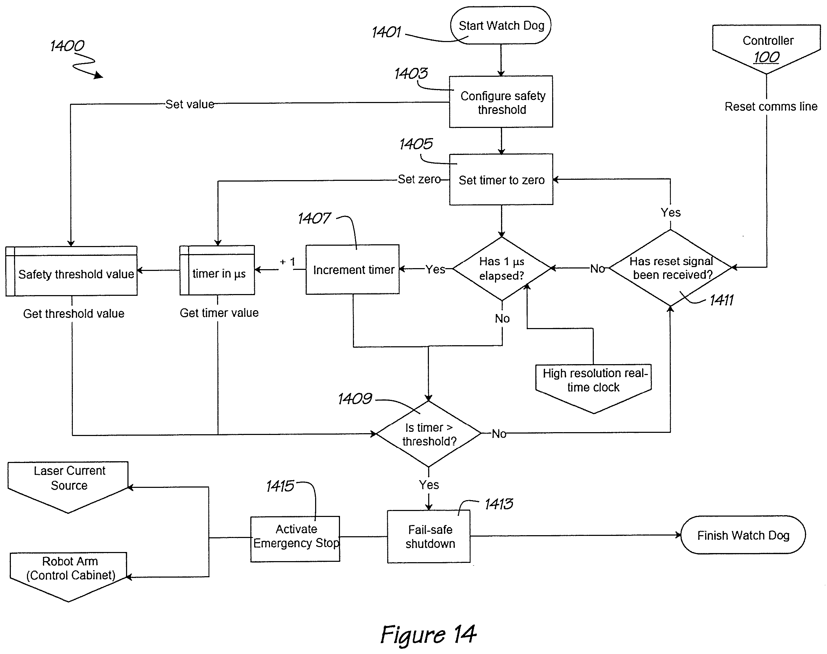

**Figure 14** of the drawings depicts a flow chart of a method **1400** of actions taken during a watch dog (hardware failsafe) procedure of the system **10.**

The watch dog **175** is started **1401** by the controller **100** on initialization of the system **10.** It is configured **1403** with a safety threshold in microseconds, and the timer is reset to zero **1405.**

For every reading from the high resolution real-time clock, the amount of time that has elapsed is incrementally added **1407** to the timer. The timer represents the number of microseconds that has elapsed since the system **10** last reported itself in a known, good state.

The timer is compared **1409** to the safety threshold value.

If it has not exceeded the threshold, then it will loop back to seeing if it has received **1411** a timer reset signal from the controller **100.** When a reset signal is received the timer is set back to zero.

If it exceeds the threshold, then the fail-safe shutdown process is initiated **1413.** For the system **10** of the embodiment this comprises activating an emergency stop **1415** on the two components that have a critical risk: the laser current source **131** and the robot arm **105** (robot arm controller **106).** Such action will immediately stop the laser light beam **30** from firing and the robot arm **105** from moving.

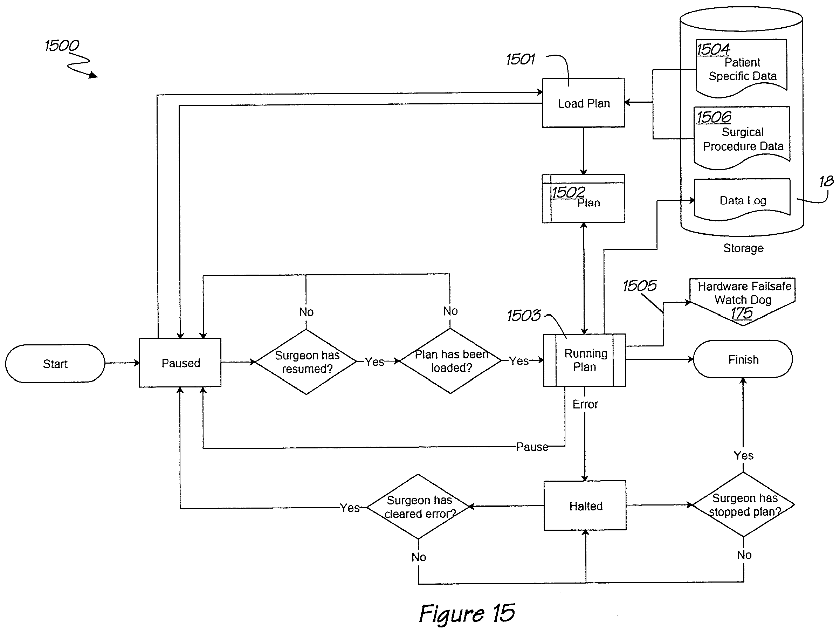

**Figure 15** depicts a flow chart of a method **1500** of actions performed by the system coordinator **200.**

The system coordinator **200** starts in a paused state.

The surgeon **16** will direct the system coordinator **200** to load **1501** a plan **1502** for action(s) to be performed from storage **18.** The plan **1502** consists of patient specific data **1504** and surgical procedure data **1506.**

If the surgeon **16** chooses to resume the system **10**, and there has been a plan loaded, the plan **1502** will be executed **1503** by the running plan process.

During the running plan process **1503,** the hardware failsafe watch dog **175** is continually sent **1505** an "everything is ok" signal that prevents the fail-safe from activating. Should the system coordinator **200** fail to send the signal within a threshold timeout then the system **10** is assumed to be in an unknown state and the hardware is set to a safe state as hereinbefore described above with reference to method **1400.**

In the embodiment, the processing of input performed by the system coordinator **200** comprises an analysis of the input and a making of a decision on the basis of the analysis. Once a decision has been made, the system coordinator **200** is operable to initiate an action on the basis of the decision to control the robot arm **105** and the laser to work the biological tissue **12.**

As part of the analysis, the system coordinator **200** is operable to generate based on input, and/or receive as input, at least one first representation of the biological tissue **12.** In the embodiment, the at least one first representation of the biological tissue **12** comprises a representation of a first or pre-action (initial) state of the biological tissue **12** corresponding to an actual state of the biological tissue **12** prior to the at least one action of work being performed thereon. The at least one first representation of the biological tissue **12** includes parameters that describe or provide an indication of one or more characteristics of the biological tissue **12.**

In the embodiment, the at least one first representation of the biological tissue **12** comprises a map or model of the pre-action state of the biological tissue **12.**

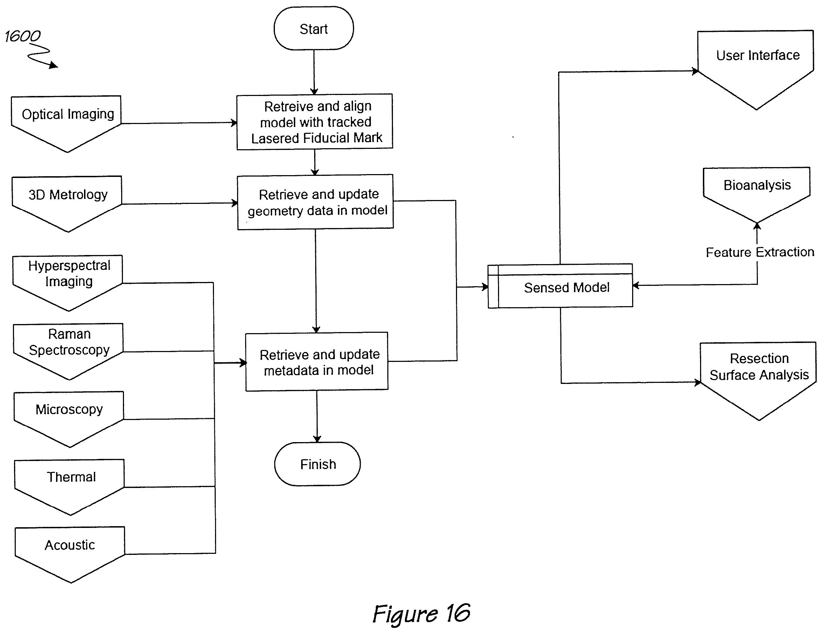

**Figure 16** depicts a flow chart of a method **1600** of actions performed during a mapping operation of the system **10** undertaken to generate the map.

The system **10** of the embodiment needs to understand the 3D surface and composition of the biological tissue **12** and surrounding environment. This representation of the physical reality is used by several subsystems of the system **10** to reason and plan the actions that will be performed. It is important that the accuracy of the mapping is sufficient for the system **10** to be able to safely and correctly perform resections of hard biological tissue.

There are several ways the system **10** senses the required mapping information, as follows.

As described herein, the system **10** is operable to collate all of this information and incorporate it into a cohesive model that parts of the system **10** can reason about given their own requirements.

The at least one first representation may comprise a plan for the work to be performed, which may comprise a setting of parameters defining one or more features or characteristics of the work.

The system **10** of the embodiment is operable to use a planning component to coordinate the actions required to correctly perform the necessary action, comprising resections of the surgical procedure in the embodiment.

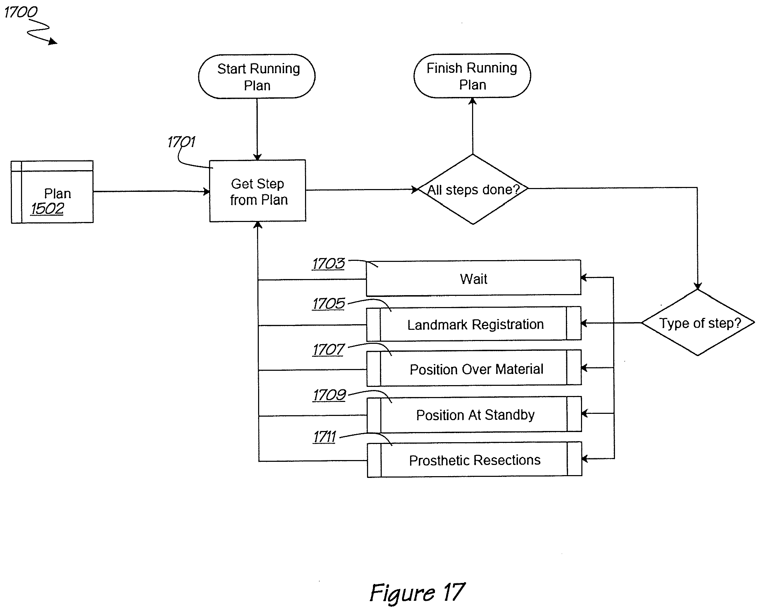

**Figure 17** depicts a flow chart of a method **1700** of actions performed during a planning operation of the system **10.**

The planning process controls the sequential actions that occur when following a plan **1502** that was loaded from storage **18.**

The next step is retrieved **1701** from the plan and the matching step process is performed.

This is repeated until all the steps of the plan have been completed. In the embodiment, steps of the plan may include: a wait operation step **1703;** a landmark registration operation step **1705;** a position over material operation step **1707;** a position at standby operation step **179;** and a prosthetic resections operation step **1711.**

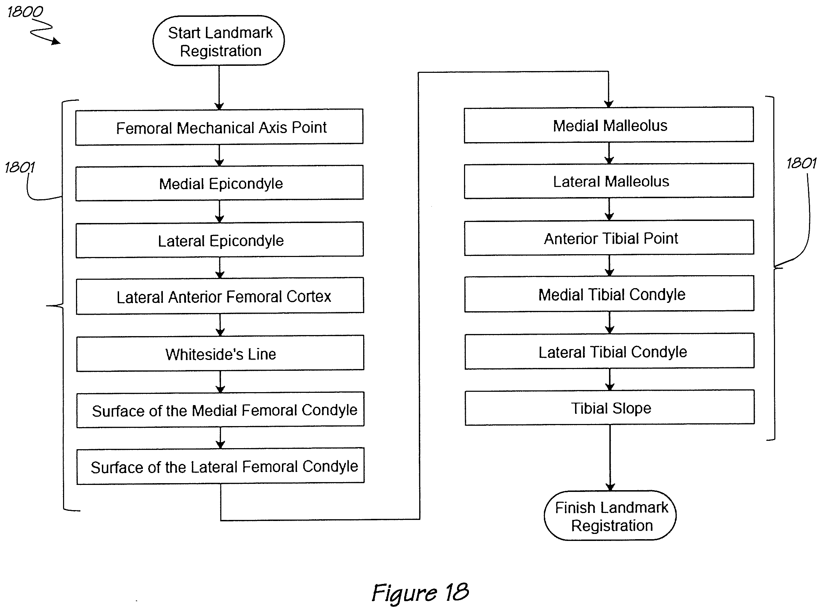

**Figure 18** depicts a flow chart of a method **1800** of actions performed during a landmark registration operation of the system **10.**

The landmark registration process involves a step-by-step registration of points in three dimensional space by the surgeon **16** using a tool with an attached fiducial marker **70.** The position sensor **170** determines the position of the working end of the tool when each point is prompted for by the user interface **21.** In the embodiment in the context of a surgical procedure to the knee of patient **14,** the points include the following sites **1801** in sequence between start of the landmark registration process and the end of the landmark registration process: Femoral Mechanical Axis Point; Medial Epicondyle; Lateral Epicondyle; Lateral Anterior Femoral Cortex; Whiteside's Line; Surface of the Medial Femoral Condyle; Surface of the Lateral Femoral Condyle; Medial Malleolus; Lateral Malleolus; Anterior Tibial Point; Medial Tibial Condyle; Lateral Tibial Condyle; and Tibial Slope.

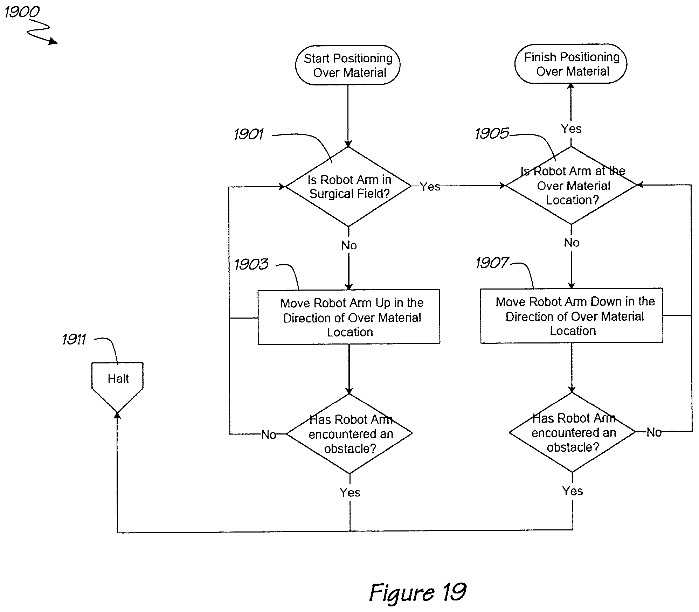

**Figure 19** depicts a flow chart of a method **1900** of actions performed during a position-over-material operation of the system **10.**

The position-over-material process **1900** ensures that the robot arm **105** is safely manoeuvred to the correct relative position above the biological tissue **12.**

Firstly a check **1901** is performed to determine whether the robot arm **105** is within a surgical field comprising prescribed conical volume of space originating at the biological tissue **12** and extending vertically such that sides of the cone are at 45°. If the robot arm **105** is not within the surgical field, the robot arm **105** is repositioned **1903** by raising the end effector **28** up to the extension limits while moving it laterally towards the biological tissue **12** until it is within the surgical field positioned over the biological tissue material to be worked upon.

Once the robot arm **105** is within the surgical field **1905** it will start to lower **1907** the end effector **28** towards the correct position above the biological tissue **12.** Once it is in the correct position it will stop **1911** moving the robot arm **105.**

Should the robot arm **105** encounter an obstacle at any stage, by virtue of being detected via increased torque forces on one or more joints of the manipulator **23,** or by virtue of operation of one or more pressure sensitive pads on the outside of the segments of the manipulator **23,** for example, then it will halt **1911** to prevent it from impacting on personnel or other objects in the environment.

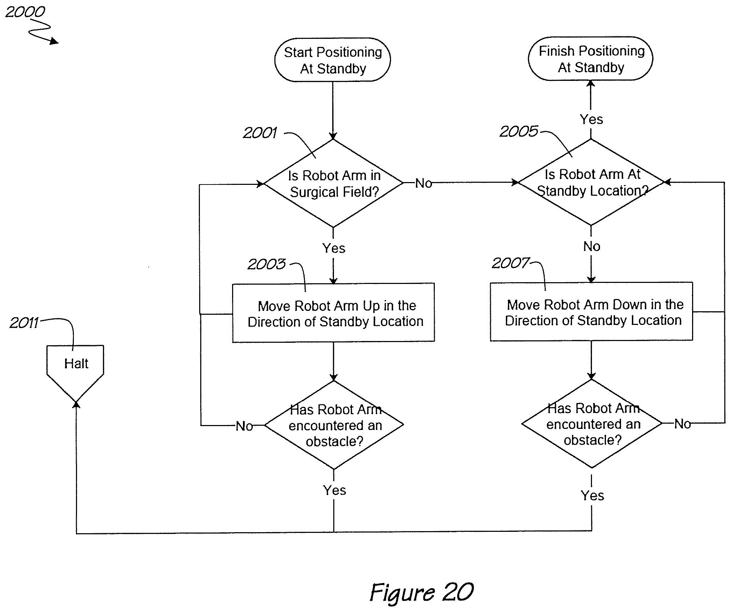

**Figure 20** depicts a flow chart of a method **2000** of actions performed during a position at standby operation of the system **10.**

This ensures that the robot arm **105** is safely manoeuvred away from the biological tissue **12** to a standby location.

If the robot arm **105** is within the surgical field **2001** then the end effector **28** is repositioned **2005** by raising it up towards the extension limits of the robot arm **105** while moving it laterally in the direction of the standby location. In the embodiment, the standby location is the position of the robot arm **105** over the base unit **27** such that it cannot interfere or be an obstacle to any reasonable actions that are performed in the surgical workspace.

Once the robot arm **105** is outside the surgical field over the standby location **2005,** the end effector **28** is lowered **2007** towards the standby location while continuing to laterally move in that same direction until it has arrived. Then it will stop **2011.**

In the same manner as the flow chart of a method **2100** of actions performed of method **1900** depicted in **Figure 19****,** should the robot arm **105** encounter an obstacle at any stage it will halt **2011.**

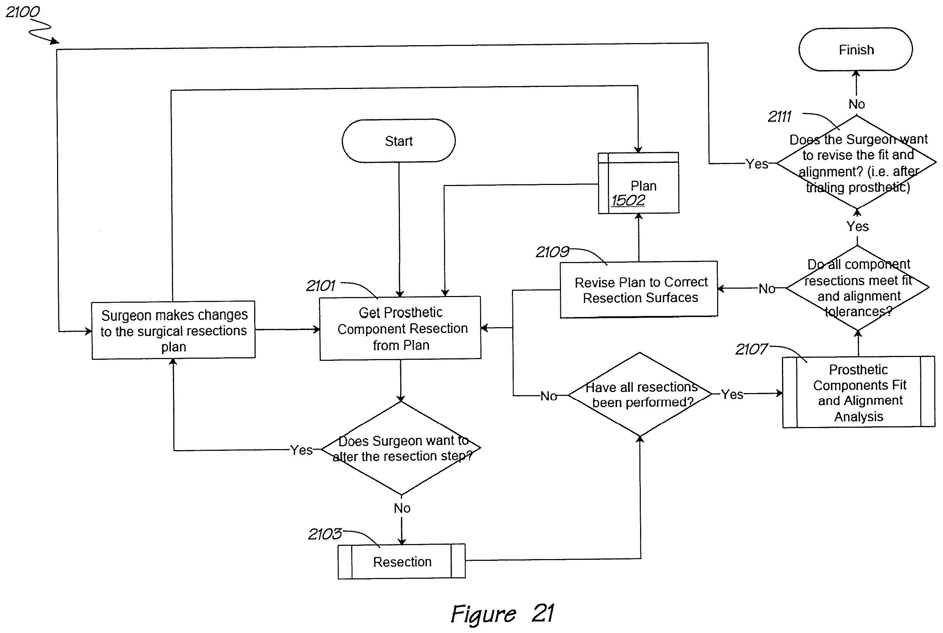

**Figure 21** depicts a flow chart of a method **2100** of actions performed during a prosthetic resections operation of the system **10.**

The process **2100** retrieves **2101** the next prosthetic component resection from the plan **1502.** The order of the resections in the plan has been determined by the surgical procedure data as seen in **Figure 17****.**

The surgeon **16** is given the opportunity **2103** of altering or changing the planned resection prior to the system **10** actioning it. This may, for example, adjust the parameters of the resection, or select a different resection to be actioned next.

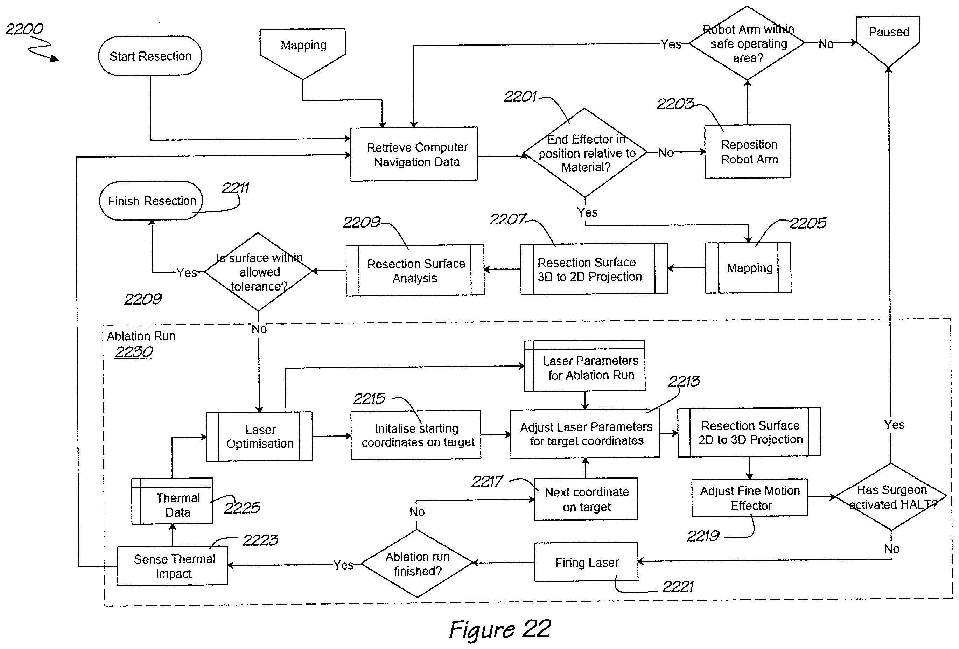

If the surgeon **16** does not want to alter the resection step then the system **10** is operable to perform the resection **2105** as will be described in further detail referring to **Figure 22****.**

If all the resections in the plan have not been performed yet, the process will retrieve **2101** the next resection from the plan until all resections have been performed.

Once all the resections in the plan have been performed, the system **10** is operable to perform a prosthetic components fit and alignment analysis **2107.**

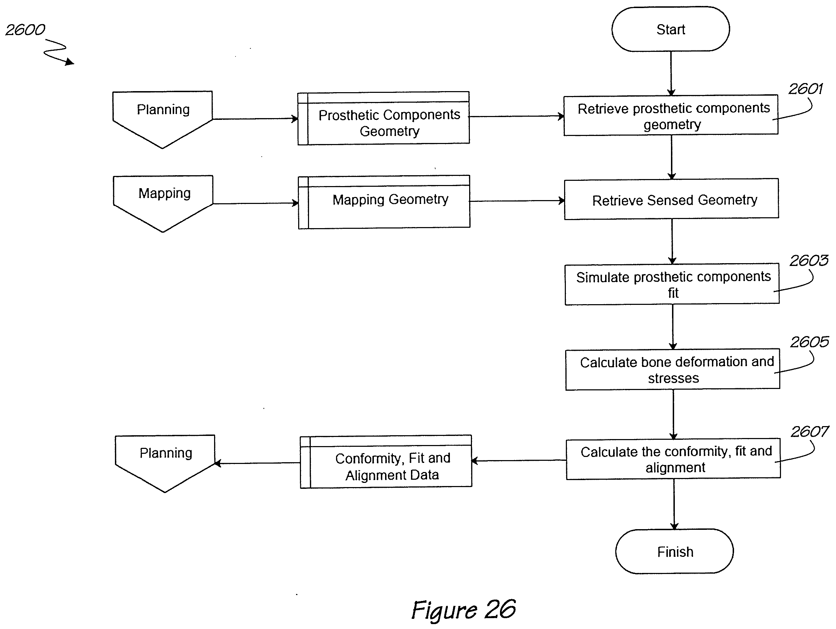

In this embodiment, this assessment is undertaken by the system **10** performing a prosthetic components fit and alignment analysis as will be described in further detail referring to **Figure 26****.**

If all the component resections do not meet the fit and alignment tolerances for sufficient positioning then the system **10** is operable to change **2109** the plan to perform revision resections steps to correct for the discrepancy between the ideal fit and alignment and the current geometric fit and alignment.

If the prosthetic components fit and alignment analysis is sufficient then the surgeon **16** is provided an opportunity to independently review **2111** the analysis and optionally make revisions to the resection plan to affect a variation on fit and alignment. This may be important after trialling the prosthetic and determining that the flexion and extension of the joint do not provide the correct clearances and adjustment resections or different sized prosthetic component(s) are required, for example.

**Figure 22** depicts a flow chart of a method **2200** of actions performed during a resection operation of the system **10.**

In the embodiment, a planned state of the biological tissue **12** after the at least one action of work has been performed thereon comprises an ideal plane or mesh in 3D space that needs to be exposed on the biological tissue **12** via subtractive ablation. The resection process is given the ideal plane or mesh for use in the processing.

The process confirms **2201** that the robot arm **105** is positioned at the correct location above the biological tissue **12.** Given that the biological tissue **12** is not held exactly still it is necessary to constantly check and readjust **2203** the absolute position of the end effector **28** as herein described to maintain the same relative location. The location of the biological tissue **12** is retrieved from the mapping component, which in turn had retrieved the positions of fiducial markers **70** via the position sensor controller **171.**

A 'safe operating area' is a virtual region that the robot arm **105** is not allowed to leave. Should the robot arm **105** attempt to reposition outside this safe operating area then the system **10** is operable to be put into a paused state until released by the user. This is an added safety mechanism to prevent the robot arm **105** from making physical contact, with surgical staff for example.

At the correct relative location, the system **10** is operable to scan the geometry of the target using the 3D metrology sensor **190.** Several sensors of the sets of sensors **101** and **102** then scan **2205** the biological tissue **12** to determine the composition thereof. These sensors include those for hyperspectral imaging, Raman spectroscopy, thermal imaging, microscopy, acoustic and optical imaging. The collective results of the scans are analysed, combined and stored by the system **10** and made available by the mapping component.

The resection surface is converted **2207** from 3D information into 2D maps to extract the data unnecessary for processing the surface topography and composition in the resection surface analysis. The geometry is reduced to a 2D height map with associated hyperspectral and thermal 2D maps overlays in the same coordinate space.

The process analyses **2209** the desired resection geometry with the height map and mapping to determine the current conformity of the surface to the tolerances required for an accurately completed resection. If the surface is complete within tolerance then this resection is done **2211.**

Otherwise, the system **10** is operable to calculate an ablation run **2230** - a series of laser ablations that will reduce the surface by a layer of material.

The system **10** is operable to perform an analysis of the extent to which the post-action state of the biological tissue **12** aligns with the planned state of the biological tissue **12** to determine the parameters of the ablation run required.

Particularly, in the embodiment, the process analyses **2209** the resection volume in conjunction with the mapping composition results and 3D surface data, composition data and any thermal data retrieved from prior ablations to determine the current conformity of the sensed geometry to an optimal set of parameters for the laser light beam **30** to perform an ablation run **2230** to safely and efficiently reduce the surface of the biological tissue **12** without burning or causing necrosis.

As part of the analysis, the system coordinator **200** (of **Figure 13****)** is operable to generate based on input, and/or receive as input, at least one third representation of the biological tissue **12.** In the embodiment, the at least one third representation of the biological tissue **12** comprises a representation of a state of the biological tissue **12** corresponding to an actual post-action or final state of the biological tissue **12** after the at least one action of work has been performed thereon (evaluation **2909).** The at least one third representation of the biological tissue **12** includes parameters that describe or provide an indication of one or more characteristics of the biological tissue **12** and, in the embodiment, comprises a map or model of the post-action state of the biological tissue **12.**

The system **10** is operable to initialize the starting coordinates **2215** on the target biological tissue **12** for the beginning of the ablation run.

During the ablation run the system **10** is operable to adjust **2213** the laser parameters such as the current and pulse duration parameters in real-time to the pre-calculated values for each target coordinates.

The process calculates the position of the 2D map target coordinates in 3D space.

The fine motion means, in the preferred embodiment a scan head **117,** is operable to reposition the direction of the laser light beam **30** to point to the target on the surface of the biological tissue **12.** Each firing sequence of the laser light beam **30** needs to be made to a point on the biological tissue **12** to micron-scale precision in the embodiment.

The system **10** is operable to check to ensure that the surgeon **16** has not halted the system **10** prior to every firing of the laser light beam **30** in the embodiment.

The laser current source **131** is directed to fire the laser light beam **30** using the current parameter settings.

If the ablation run has not been completed yet, the next coordinates in the map are selected **2217,** the laser parameters are adjusted **2213** and the system **10** repeats the repositioning **2219** and firing **2221** of the laser light beam **30.**

Once the ablation run has completed, the surface is sensed **2223** with the thermal sensor 184 for heat impact. The thermal data **2225** arising from such sensing is made available to the laser optimisation component for future ablation runs.

The location of the biological tissue **12** is checked for positioning the end effector **28** and the process of mapping, analysing and ablating is repeated.

Regarding robot motion, the system **10** is operable to position the sensors, laser optics and ancillary tools in the end effector **28** above and at a set relative distance from the biological tissue **12.**

The positioning depends on the joint angles of the robot arm **105.** The calculation of the joint angles for the end position and all transition positions in-between involves inverse kinematics.

Preferably, the system **10** will rely on and use provided inverse-kinematics infrastructure in the robot arm **105** to determine the joint angles and movement required to transition the end effector **28** from one Cartesian point to another. This will result in the robot motion component being a façade for that existing functionality.

Should the robot arm controller **106** not provide the required functionality then it will be necessary to use an existing or custom library that supports the inverse kinematic calculations.

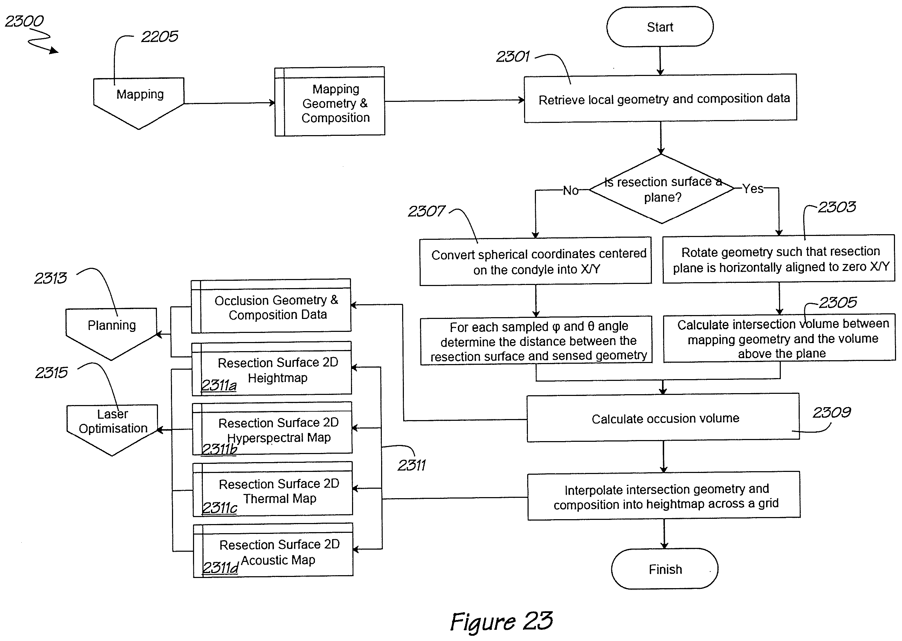

**Figure 23** depicts a flow chart of a method **2300** of actions performed during a resection surface 3D to 2D projection.

When a resection is being performed, the system **10** is operable to maintain an understanding of the sensed geometry as it applies to the subtractive process of reducing biological tissue **12** to the desired geometry.

The mapping component holds the mesh and other ancillary data that describes the geometry and composition of biological tissue **12.** The projection process uses that information to create a specialised derived data source orientated for the resection parameters.

The process is operable to retrieve **2301** the geometry and composition data from the mapping component.

Where the resection surface is a plane, the mapping geometry is rotated **2303** around the centre of the transepicondylar axis such that the coordinates of the resection plane is aligned to the zero axis of the X and Y coordinates (where Z is up/down).

The intersection volume between the mapping geometry and the positive Z area above the X/Y plane is calculated **2205.** The part of the volume that is bone is the section that needs to be subtractively ablated. The remaining volume is potentially occluding material that needs to remain and should not be ablated, nor should it be in line with a ray projection calculation between the laser beam origin and any target point for ablation.

As the bone volume X/Y plane has been aligned to the resection plane, the Z value can simply be viewed as a mesh at varying heights above the desired flat end result. A height map can be interpolated from the mesh by applying a grid with a resolution of the minimum scale required to perform the flat plane ablation.

Where the resection surface is not a plane, the geometric mesh is calculated **2307** in spherical coordinates from the centre of the condyle (as defined as ¼ or ¾ position on the transepicondylar axis depending on which condyle the resection surface is located).

The φ and θ angle is sampled at sufficiently small degrees such that, at the average distance to the surface, the grid spacing is around 50 microns. In essence, this allows for the sampled φ to become the x axis on a 2D map, and for the θ angle to become the y axis. The ideal resection surface is considered to be at zero height and any existing biological tissue 12 above that is treated as positive height value. This newly created height map can then be analysed and processed the same as if the resection was occurring on a plane.

The process is then operable to determine **2309** the occlusion geometry by removing any geometry that does not exist within the intersection of the mapping geometry and an inclusion shape.

For the femoral head the inclusion shape comprises the intersection of:
(1) two ovals centred on the ¼ and % position of the transepicondylar axis with a width radius (along the axis) of ¼ the transepicondylar axis and a height radius of the transepicondylar axis; and
(2) a solid cylinder with the long axis and height of the transepicondylar axis and a radius of ¾ the transepicondylar axis.

For the tibial plateau the inclusion shape comprises a solid cylinder with the long axis in line with the cortex with a radius of ½ the distance of the width of the plateau.

The process is then operable to create a set of 2D maps **2311** in the same coordinate space for the height, hyperspectral values and thermal values. This reduced dimensionality data is used by various components of the system 10 to analyse the resection progress and determine the laser optimisation for ablation.

As part of the analysis, the system coordinator 200 (of **Figure** 13) is operable to compare the representation of the post-action state of the biological tissue 12 with the representation of the planned state of the biological tissue 12 to assess the extent to which the state of the biological tissue 12 after the at least one action of work has been performed thereon aligns with the planned state of the biological tissue 12, (i.e. mapping step **2205** of **Figure** 22 and method **2300** of **Figure** 23) and thereby assess the extent to which the action has been formed successfully. A measure of success is provided based on the extent to which the representation of the post-action state of the biological tissue 12 fits and aligns with the representation of the planned state of the biological tissue 12 to prescribed tolerances. This process can be summarised as determining how the sensed reality should be represented internally in a useful way for processing and determining the laser ablation objectives. In **Figure** 23 it can be seen that there are four (4) representations **2311** further used by the Planning **2313** and Laser Optimisation **2315** processes including: the "Re-section surface 2D Heightmap" **2311a,** "Re-section Surface 2D Hyperspectral Map" **2311b,** "Re-section Surface 2D Thermal Map" **2311c,** and "Re-section Surface 2D Acoustic Map" **2311d.**

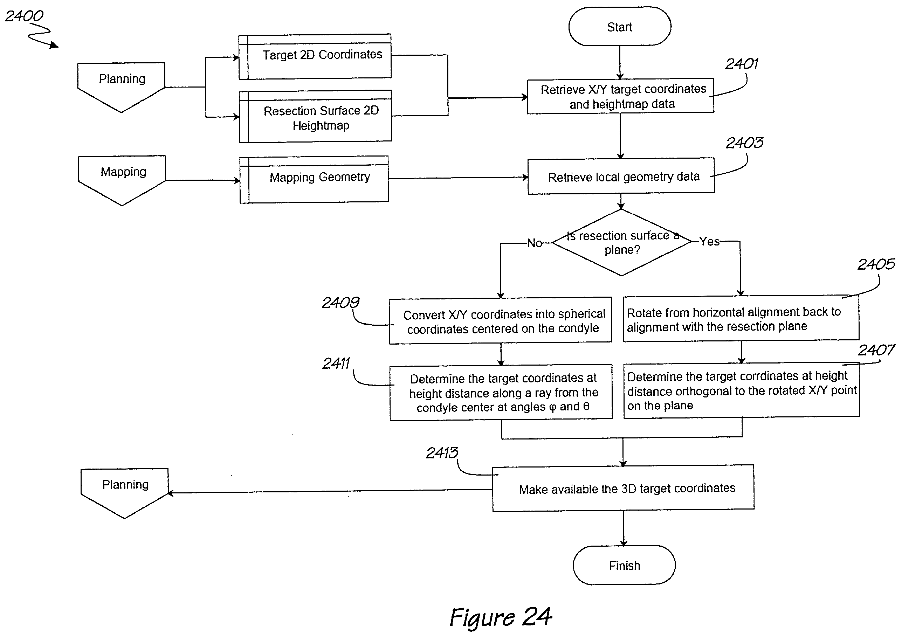

**Figure 24** depicts a flow chart of a method **2400** of actions performed to reverse the 3D to 2D projection that was used to reduce the dimensionality of the data. The system 10 requires the 3D location of each target coordinate on the 2D map for positioning the laser light beam 30 to ablate the correct biological tissue 12.

The process is operable to retrieve **2401** the X/Y target coordinates from an ablation run and the height map data in the 2D map. The local geometry data is also retrieved **2403** from the mapping component.

If the resection surface is a plane then the X/Y target coordinates are rotated **2405** from the horizontal alignment back into alignment with the resection plane. The 3D target coordinates can then be determined **2407** at the height distance orthogonal to the now rotated plane.

If the resection surface is not a plane the process is operable to convert **2409** the X/Y coordinates into φ and θ angle spherical coordinates centred on the condyle. The 3D target coordinates can then be determined **2411** at the height distance along a ray from the condyle centre at the angles φ and θ.

The resulting 3D coordinates are made available **2413** such that the system 10 can then target the biological tissue 12 at the correct location in world coordinates.

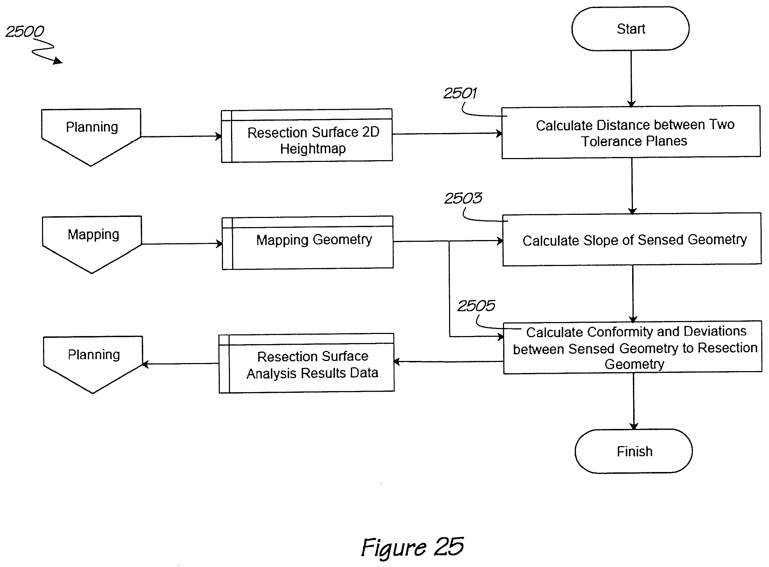

**Figure 25** depicts a flow chart of a method **2500** of actions performed during a resection surface analysis operation of the system 10.

The system **10** is operable to calculate **2501** the distance between two tolerance planes that enclose all the geometry of the surface. This provides one quantification of the overall flatness of the geometry.

The system **10** is also operable to calculate **2503** the slope of the geometry by best fitting a plane to the vertices. This slope information is important to the overall alignment of the prosthetic component.

The system **10** is operable to then quantify **2505** the specific deviations and conformity that the surface geometry has compared to the ideal geometry of the surface plan. This includes the primary profile, roughness profile, waviness profile and shape profile which may be determined using standard methods of surface attribute analysis.

As part of the analysis, the system coordinator **200** is operable to generate based on input, and/or receive as input, at least one second representation of the biological tissue 12. In the embodiment, the at least one second representation of the biological tissue **12** comprises a representation of a state of the biological tissue **12** corresponding to a planned or intended state of the biological tissue **12** after the at least one action of work has been performed thereon. The at least one second representation of the biological tissue **12** includes parameters that describe or provide an indication of one or more characteristics of the biological tissue **12** and, in the embodiment, comprises a map or model of the planned state of the biological tissue 12.

The resulting set of analysis data is provided to the planning system for use in prosthetic component fit and alignment analysis.

**Figure 26** depicts a flow chart of a method **2600** of actions performed during a prosthetic components fit and alignment analysis operation of the system 10.

The prosthetic components geometry is retrieved **2601** from the loaded plan for processing against the retrieved mapping geometry. The system **10** is operable to simulate **2603** the prosthetic component onto the mapping geometry by way of finite element method to detect/calculate **2605** the stresses and deformations that are likely to result to the fit and subsequent alignment of the component to the mechanical axis of the patient 14.

The quantified **2607** conformity, fit and alignment data is made available to the system 10 for automated and surgeon **16** decision making on the resections being performed. The results are also presented to the surgeon **16** via the display **20.**

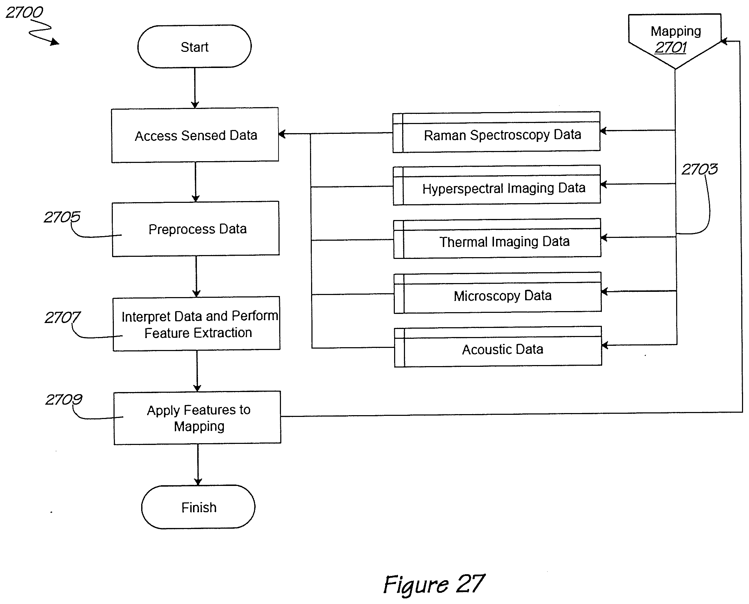

**Figure 27** depicts a flow chart of a method **2700** of actions performed during a bioanalysis operation of the system 10 where the sensing information is analysed for composition features of the biological tissue **12.** This helps to prevent the system **10** from targeting soft tissue or otherwise causing collateral damage to parts of the patient **14** that should not be resected.

The mapping component **2701** has already aligned all the sensor data **2703** to the geometry surface to account for the sensors being placed at different viewpoints from the biological tissue **12.**

In embodiments of the invention, the data can be pre-processed **2705** to remove noise and artefacts in the sensed information that would obscure the feature detection.

The system 10 is operable to perform feature extraction **2707** on the data by comparing the data signal with known signatures that have been determined from prior testing. This can be done for the same target location across the various measurement sensors of the sets of sensors **101** and **102** to determine a prediction of the composition for each point.

The system **10** is operable to then apply **2709** the bioanalysis predictions back to the mapping component to provide additional metadata for use by other parts of the system 10 to guide decisions and actions.

**Figure 28** depicts a flow chart of a method **2800** of actions performed during an acoustic sound analysis operation of the system **10.**

The bioanalysis component also uses the acoustic sensor **178** to determine if the sound of an ablation has been performed on hard biological tissue.

The sound profile produced by laser ablating bone is distinct from the sounds produced by other materials. The system 10 is operable to utilise this to increase the overall safety during an ablation run. Method **2800** comprises the step of receiving **2801** sound data **177** picked up by an acoustic sensor **178** from the environment and around the biological tissue **12** at the working site and analysing **2803** the sound data to determine whether the received sound data matches that expected when the laser impact a bone material. When an unexpected sound is received and analysed, the system 10 is operable to halt **2805** the ablation. Due to the speed of the laser pulses this may not prevent a few more laser bursts from striking a non-bone surface until the processing of the prior sound profile has completed, but can prevent further damage.

The system 10 is operable to detect if the laser light beam 30 has just been fired and access the acoustic sensor 178 sound data comprising a sound wave. The sound wave is analysed to detect if it matches, by way of distinct frequency and amplitude, the profile of hard biological tissue being ablated.

If the sound does not match then the system 10 is signalled to halt operation.

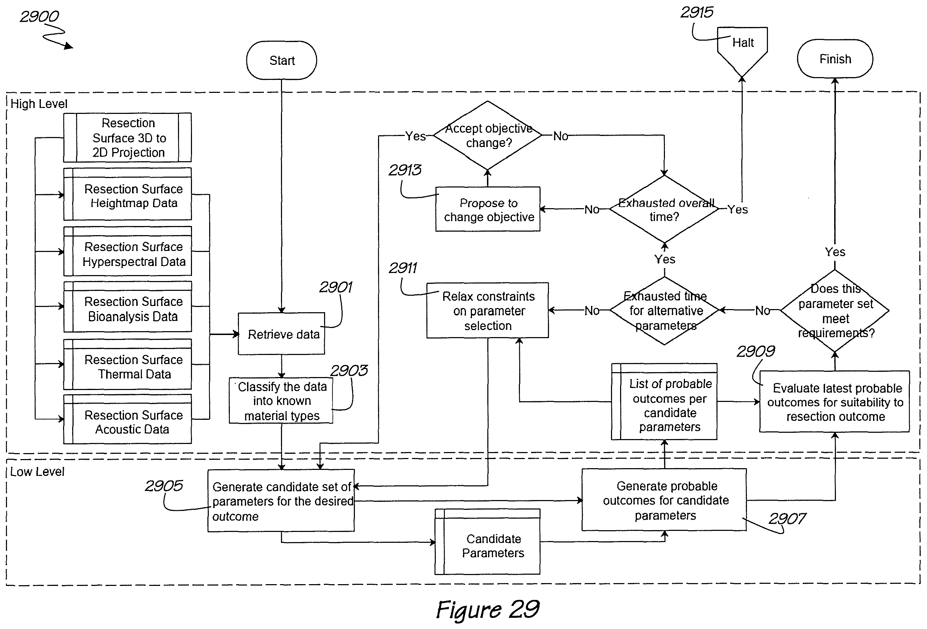

**Figure 29** depicts a flow chart of a method **2900** of actions performed during a laser optimisation operation of the system 10.

The purpose of this component is to perform risk-sensitive decision-making for autonomous control of the laser parameters in the minimally-observable environment.

The ablative laser is controlled through several co-dependent parameters that influence how it interacts with different materials. The task of laser-based machining is complex as it involves tuning these parameters to suit the material being ablated. This material differs between patients depending on the type of bone and its composition (mineral, collagen and water content), as well as factors such as gender, age and ethnicity. The laser optimisation determines the optimal set of parameters for the laser light beam 30 to safely ablate hard tissue. In addition, the system 10 is operable to quantify its level of confidence at every stage of the decision-making process and has the ability to output human-intelligible diagnostic information.

The laser optimisation component retrieves **2901** data from the resection surface analysis component. This has been pre-processed into a series of 2D maps: height, hyperspectral, bioanalysis, thermal and acoustic. The 2D map is either an oriented resection plane or a map from spherical coordinates for a resection surface.

From this base of data, the component can classify **2903** the data into known material types. As a low level function, a set of candidate laser parameters is generated **2905** that is predicted to result in the desired outcome. These are tested in a model to determine **2907** the most likely outcome for those candidates.

The suitability of those outcomes is then evaluated **2909.** In some instances, there could be undesirable results predicted, so the component has the option to relax **2911** some of the constraints on parameter selection before generating **2905** a new set of parameters for testing.

In cases where the component has exceeded the time period to trial alternatives, the objective can be changed **2913** to facilitate parameter selection that could achieve a partial solution rather than have no solution.

If the component exhausts the time period for alternative parameters and no solutions have been reached, then the system **10** is placed in halt **2915.**

Safety, monitoring and verification operations of the system **10** comprise:
- correct cutting position;
- expected tissue type; and
- expected effects (thermal, stress, material removal).

Outcomes and results that may arise from operation of the system **10** include:
- efficiency, including in the cutting/shaping rate;
- geometric effects, including in the cut physical attributes; and
- side effects, including thermal effects:

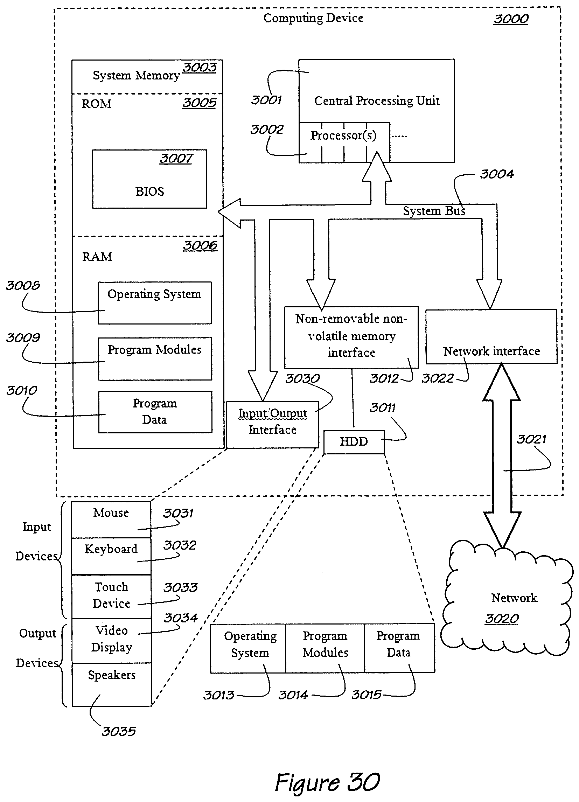

The methods **1400** to **2900** (and associated sub methods depicted in **Figures 14 to 29** may be implemented using a computing device / computer system **3000,** such as that shown in **Figure 30** wherein the processes of **Figures 14** to **29** may be implemented as software, such as one or more application programs executable within the computing device **3000.** In particular, the steps of the methods disclosed herein are effected by instructions in the software that are carried out within the computer system **3000.** The instructions may be formed as one or more code modules, each for performing one or more particular tasks. The software may also be divided into two separate parts, in which a first part and the corresponding code modules performs the described methods and a second part and the corresponding code modules manage a user interface between the first part and the user. The software may be stored in a computer readable medium, including the storage devices described below, for example. The software is loaded into the computer system **3000** from the computer readable medium, and then executed by the computer system 3000. A computer readable medium having such software or computer program recorded on it is a computer program product. The use of the computer program product in the computer system 3000 preferably effects an advantageous apparatus for laser shaping of biological tissue including bone.

With reference to Figure 30, an exemplary computing device 3000 is illustrated. The example computing device 3000 can include, but is not limited to, one or more central processing units (CPUs) 3001 comprising one or more processors 3002, a system memory 3003, and a system bus 3004 that couples various system components including the system memory 3003 to the processing unit 3001. The system bus 3004may be any of several types of bus structures including a memory bus or memory controller, a peripheral bus, and a local bus using any of a variety of bus architectures.

The computing device 3000 also typically includes computer readable media, which can include any available media that can be accessed by computing device 3000 and includes both volatile and non-volatile media and removable and non-removable media. By way of example, and not limitation, computer readable media may comprise computer storage media and communication media. Computer storage media includes media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can be accessed by the computing device 3000. Communication media typically embodies computer readable instructions, data structures, program modules or other data in a modulated data signal such as a carrier wave or other transport mechanism and includes any information delivery media. By way of example, and not limitation, communication media includes wired media such as a wired network or direct-wired connection, and wireless media such as acoustic, RF, infrared and other wireless media. Combinations of the any of the above should also be included within the scope of computer readable media.

The system memory 3003 includes computer storage media in the form of volatile and/or non-volatile memory such as read only memory (ROM) 3005 and random access memory (RAM) 3006. A basic input/output system 3007 (BIOS), containing the basic routines that help to transfer information between elements within computing device 3000, such as during start-up, is typically stored in ROM **3005**. RAM 3006-typically contains data and/or program modules that are immediately accessible to and/or presently being operated on by processing unit 3001. By way of example, and not limitation, Figure 30 illustrates an operating system 3008, other program modules 3009, and program data 3010.

The computing device 3000 may also include other removable/non-removable, volatile/non-volatile computer storage media. By way of example only, Figure 30 illustrates a hard disk drive 3011 that reads from or writes to non-removable, non-volatile magnetic media. Other removable/non-removable, volatile/non-volatile computer storage media that can be used with the example computing device include, but are not limited to, magnetic tape cassettes, flash memory cards, digital versatile disks, digital video tape, solid state RAM, solid state ROM, and the like. The hard disk drive 3011 is typically connected to the system bus 3004 through a non-removable memory interface such as interface 3012.

The drives and their associated computer storage media discussed above and illustrated in Figure 30, provide storage of computer readable instructions, data structures, program modules and other data for the computing device 3000. In Figure 30, for example, hard disk drive 3011 is illustrated as storing an operating system 3013, other program modules 3014, and program data 3015. Note that these components can either be the same as or different from operating system 3008, other program modules 3009 and program data 3010. Operating system 3013, other program modules 3014 and program data 3015 are given different numbers hereto illustrate that, at a minimum, they are different copies.

The computing device also includes one or more input/output (I/O) interfaces 3030 connected to the system bus 3004 including an audio-video interface that couples to output devices including one or more of a video display 3034 and loudspeakers 3035. Input/output interface(s) 3030 also couple(s) to one or more input devices including, for example a mouse 3031, keyboard 3032 or touch sensitive device 3033 such as for example a smartphone or tablet device.

Of relevance to the descriptions below, the computing device 3000 may operate in a networked environment using logical connections to one or more remote computers. For simplicity of illustration, the computing device **3000** is shown in **Figure 30** to be connected to a network **3020** that is not limited to any particular network or networking protocols, but which may include, for example Ethernet, Bluetooth or IEEE 802.X wireless protocols. The logical connection depicted in **Figure 30** is a general network connection **3021** that can be a local area network (LAN), a wide area network (WAN) or other network, for example, the internet. The computing device **3000** is connected to the general network connection **3021** through a network interface or adapter **3022** which is, in turn, connected to the system bus **3004.** In a networked environment, program modules depicted relative to the computing device **3000,** or portions or peripherals thereof, may be stored in the memory of one or more other computing devices that are communicatively coupled to the computing device **3000** through the general network connection **3021.** It will be appreciated that the network connections shown are examples only and other means of establishing a communications link between computing devices may be used.

In embodiments of the invention, one or more of the described, additional and/or alternative operations performed by the system 10 occur semi-automatically or automatically, without requiring human intervention.

It can be appreciated that in the implementation described a laser is coupled with a robot and the couple is integrated with optical and mechanical sensors to provide a closed loop subtractive laser-assisted machining of bone system (machine), allowing for computationally derived process parameters for design and selection of operating parameters during real orthopaedic surgery in a clinical environment for rapid and accurate outcome.

It will be appreciated that the described embodiment of the invention provides several advantages.

The embodiment of the system 10 is capable of machining hard biological tissue with submillimetre precision, and includes features of: non-invasive real-time optical sensing; intelligent and dynamic control of laser parameters; and post-machining biological validation.

The embodiment of the invention: uses non-invasive optical sensing to ensure precision control, safety and localisation of the laser; involves the development and teaching of an artificial intelligence system to control the process of laser shaping in real-time; involves validation of the optimised laser parameters against cellular and molecular assessments; and optimises the performance of the integrated system.

Embodiments of the invention may allow for surgery with improvement in accuracy of the cut, improvement in submillimetre precision of the cut, improvement in safety due to the monitoring ability, avoiding the need of collateral damage, improvement in the biological quality of the cut surface by optimizing the parameters of the lasers using analytical and machine learning techniques.

It is anticipated that, in the first world alone, more than 2 million patients per year could benefit from technology of embodiments of the invention.

Embodiments of the invention provide for high accuracy and submillimetre precision through intelligent adaptive shaping, low thermal and biomechanical impact, vibration free shaping; and multiple redundant safety.

Embodiments of the invention provide for patient customised laser ablation that is optimised in real-time. The system of embodiments takes in continuous feedback from sensors and models and post ablation assessment (biological status of residual tissue) and adjusts laser parameters to optimum. Resulting in maximum or improved tissue removal for absolute minimal or reduced thermal impact.

Via embodiments of the system, a laser ablation can be provided that: is customisable to each patient; has no/minimal/reduced thermal impact (biological quality); is geometrically precise; has adjustable parameters in real time; and has multiple levels of safety (including guide laser, sensors, post cut sensing, and surgeon control).

The submillimetre precision, low mechanical and thermal impact afforded by embodiments of the invention may advantageously lead to a revolution in implant manufacturing, and also to a significant improvement in patient outcome and quality of life.

Embodiments of the invention may allow for minimal human orthopaedic surgery with minimal human intervention, semi- or full automation; high precision, rapid procedure, minimal invasive tissue-damage, reduced or no blood transfusion, improved implant integration, and reduced cost.

The embodiment of the invention advantageously uses a laser to ablate biological tissue, doing so quickly within the same time constraints of surgery, safely, accurately, and cleanly.

To ablate hard biological tissue quickly within the same time constraints of surgery, the described embodiment increases or maximizes the volume of material that can be removed taking into account safety and accuracy. It is capable of ablating material faster when the target location's surrounding hard biological material will be damaged and will later need to be removed in any case. It runs liquid to be applied to the tissue for cooling the tissue during the ablation procedure bundled with the cold coolant from the base unit to the end effector to cool the liquid. Laser optimization is based on the dynamics of interaction with hard biological tissue to increase or maximize rate of machining. It performs the ablation in "runs" that encapsulates a series of laser ablations in quick bursts between sensing. This may provide an optimal balance of sensing (assured) and speed.

To ablate hard biological tissue safely, the described embodiment protects the final re-sectioned tissue surface from damage (necrosis/thermal shock/cracks). This is achieved by using low laser power to ensure that the final surface is undamaged, or to reduce damage to the final surface, and by using a water spray to introduce cool water to the material to assist in laser ablation and protect the material from thermal damage. It is further achieved by protecting operating staff with a consumable shield that helps to prevent inadvertent injury or damage from objects coming into contact with the laser beam.

To ablate hard biological tissue accurately, the described embodiment uses a robotic arm to position the end effector above the target, then uses a scan head to direct the laser with greater accuracy. Additionally, it uses a 3D metrology sensor to map the geometry of the material. In this regard, it reduces the dimensionality of the 3D geometry into a series of 2D maps based on either a plane or spherical coordinate system, allowing for faster processing and an easier to understand display for the surgeon 16. All other sensor information can be overlaid on the same 2D map. Resection surface analysis allows for best fit and alignment with the patient. It uses a hyperspectral sensor to differentiate between tissue types. It uses an acoustic sensor to detect if the laser has impacted an unexpected object. It uses a lasered fiducial mark on the material to allow for highly accurate tracking (over and above what is provided with the position sensor). It provides intelligent control over the ablation process, using feedback of sensor data to refine the laser machining process and having a master system coordinator allowing for simulation, testing, and data logging (big data).

To ablate hard biological tissue cleanly, the described embodiment incorporates a particle collector into a consumable shield that acts as a vacuum bag. Additionally, it uses a series of traps and filters to store the particulate matter, and thereby saves the final HEPA filter from becoming full quickly with large scale particles.

## Claims

1. A robot-assisted laser osteotomy system (10) for shaping biological tissue (12) including bone, the system (10) comprising:
a tool (24) comprising a laser operable to perform at least one action of work comprising laser ablation;
positioning means (25) for positioning the tool (24) relative to the biological tissue (12) to perform the at least one action of work;
an input means (22);
a controller (100) to receive criteria as input via the input means (22), process, and on the basis of the processing, control the positioning means (25) and the tool (24) to work the biological tissue (12); and
storage (18) storing electronic program instructions for operation of the controller (100);
wherein:
processing of the input performed by the controller (100), under control of the electronic program instructions, comprises an analysis of the input and a making of a decision on the basis of the analysis and criteria received by the controller (100), said criteria being criteria relevant to the at least one action of work to direct the positioning means (25) and the tool (24) to work the biological tissue (12); and, once a decision has been made, the controller is configured, under control of the electronic program instructions, to initiate an action on the basis of the decision to control the positioning means and the tool to work the material;
wherein the criteria comprise: speed of the at least one action of work; accuracy of the at least one action of work; safety of the at least one action of work; and cleanliness of the at least one action of work;
wherein as at least part of the analysis, the controller (100) is operable to:
generate based on input, or receive as input, a first representation of the biological tissue (12) comprising an actual state of the biological tissue (12) after the at least one action of work has been performed thereon;
generate a second representation of the biological tissue comprising a planned state of the biological tissue (12) after the at least one action of work; and
make an assessment regarding the extent to which the action has been formed successfully by comparing the first representation of the biological tissue (12) with the second representation of the biological tissue (12) and use the assessment in the making of a decision; and
wherein the tool (24) comprising the laser further includes a sprayer (157) for spraying a liquid on the biological tissue (12) to assist in laser ablation and protect the biological tissue (12) from thermal damage to at least some extent.

2. The system according to claim 1, wherein the input means (22) comprises at least one sensor system comprising a set of sensors (101), (102), (170), (180), wherein individual sensors within the set of sensors are operable to monitor, sense, gather, or measure sensor data and/or information associated with or relating to one or more characteristics, properties and/or parameters of one or more of the system (10), the biological tissue, the surrounding environment, components, or systems or devices associated therewith or coupled thereto, wherein sensors (101), (102), (170), (180) of the set of sensors include those based on one or more of: Raman spectroscopy; hyperspectral imaging; optical imaging; thermal imaging; fluorescence spectroscopy, microscopy, acoustic, metrology, optical coherence tomography, laser power, and any non-invasive sensing.

3. The system according to claim 1, wherein operations performed by the system (10) occur either semi-automatically under control of a surgeon, or automatically without requiring human intervention.

4. The system according to claim 1, wherein the processing of the input comprises optimizing the operation of the laser based on the dynamics of interaction between a beam (30) of radiation generated by the laser and the biological tissue (12) to increase rate of the ablation.

5. The system according to claim 1, wherein beams (30) of radiation are generated by the laser in pulses, and laser pulses are batched into ablation runs comprising a pre-calculated set of pulses at different locations across the biological tissue.

6. The system according to claim 1, wherein the system (10) includes cooling means (140) for cooling one or more components and liquids of the system (10).

7. The system according to claim 1, comprising shielding for providing protection during working of the biological tissue.

8. The system according to claim 1, wherein the positioning means (25) comprises a robot arm.

9. The system according to claim 1, wherein, when the input comprises input from a 3D metrology sensor, the processing comprises using the input to map the geometry of the biological tissue (12) and the surrounding environment.

10. The system according to claim 1, wherein, when the input comprises input from an acoustic sensor, the processing comprises using the acoustic sensor input to detect if the tool (24) has worked an unintended or unexpected biological tissue (12) or material.

11. The system according to claim 1, wherein the controller (100) comprises an intelligent controller comprising Artificial Intelligence (AI) software including, but not limited to, machine perception, or machine learning.

12. The system according to claim 1, further comprising dynamic focusing optics and a dynamic focusing optics controller; the dynamic focusing optics operable to dynamically change the focal length and focus beam diameter of the laser beam (30) at a target distance determined as desirable by the controller via the dynamic focusing optics controller.

13. A computer-readable storage medium on which is stored instructions that, when executed be a computing means, causes the computing means to perform a method, the method comprising:
storing electronic program instructions for controlling a controller (100); and
controlling the controller (100) via the electronic program instructions, to:
receive input via an input means (22); and
process the input and, on the basis of the processing, control a tool (24) operable to perform at least one action of work and positioning means for positioning the tool (24) relative to the biological tissue (12) to perform the at least one action of work, to work the biological tissue (12),
wherein:
processing of input performed by the controller (100) comprises an analysis of the input and a making of a decision on the basis of the analysis and criteria received by the controller (100), said criteria being criteria relevant to the at least one action of work to direct the positioning means (25) and the tool (24) to work the biological tissue (12);
and,
once a decision has been made, the method comprises controlling the controller (100), via the electronic program instructions, to initiate an action on the basis of the decision to control the positioning means (25) and the tool (24) to work the biological tissue; wherein the criteria comprise: speed of the at least one action of work; accuracy of the at least one action of work; safety of the at least one action of work; and cleanliness of the at least one action of work; and wherein
as at least part of the analysis, the controller (100) is operable, under control of the electronic program instructions, to:
generate based on input, or receive as input, a first representation of the biological tissue (12) comprising an actual state of the biological tissue (12) after the at least one action of work has been performed thereon;
generate based on input, or receive as input, a second representation of the biological tissue (12), the other representation of the biological tissue (12) being a different representation of the biological tissue (12) and comprising a planned state of the biological tissue (12) after the at least one action of work; and
make an assessment regarding the extent to which the action has been formed successfully by comparing the first representation of the biological tissue (12) with the second representation of the biological tissue (12), and use the assessment in the making of the decision;
wherein the tool (24) further includes a sprayer (157) for spraying a liquid onto the biological tissue (12) to assist in laser ablation and protect the biological tissue (12) from thermal damage to at least some extent.

## Patentansprüche

1. Robotergestütztes Laser-Osteotomiesystem (10) zum Formen von biologischem Gewebe (12), einschließlich Knochen, wobei das System (10) umfasst:
ein Werkzeug (24), das einen Laser umfasst, der betreibbar ist, um mindestens eine Arbeitshandlung, einschließlich Laserablation, durchzuführen;
eine Positioniereinrichtung (25) zum Positionieren des Werkzeugs (24) relativ zum biologischen Gewebe (12), um die mindestens eine Arbeitshandlung auszuführen;
eine Eingabeeinrichtung (22);
einen Controller (100) zum Empfangen von Kriterien als Eingabe über die Eingabeeinrichtung (22), zum Verarbeiten dieser Kriterien und zum Steuern der Positioniereinrichtung (25) und des Werkzeugs (24) auf der Grundlage der Verarbeitung, um das biologische Gewebe (12) zu bearbeiten; und
einen Speicher (18), der elektronische Programmbefehle für den Betrieb des Controllers (100) speichert;
wobei die von dem Controller (100) unter der Steuerung der elektronischen Programmbefehle durchgeführte Verarbeitung der Eingabe eine Analyse der Eingabe und eine Entscheidungsfindung auf der Grundlage der Analyse und der von dem Controller (100) empfangenen Kriterien umfasst, wobei es sich bei den Kriterien um Kriterien handelt, die für die mindestens eine Arbeitshandlung relevant sind, um die Positioniereinrichtung (25) und das Werkzeug (24) zur Bearbeitung des biologischen Gewebes (12) anzusteuern; und sobald eine Entscheidung getroffen wurde, der Controller so ausgebildet ist, dass er unter der Steuerung der elektronischen Programmbefehle auf der Grundlage der Entscheidung eine Maßnahme einleitet, um die Positioniereinrichtung und das Werkzeug zur Bearbeitung des Materials zu steuern;
wobei die Kriterien Folgendes umfassen: Geschwindigkeit der mindestens einen Arbeitshandlung; Genauigkeit der mindestens einen Arbeitshandlung; Sicherheit der mindestens einen Arbeitshandlung; und Sauberkeit der mindestens einen Arbeitshandlung;
wobei der Controller (100) als zumindest Teil der Analyse betreibbar ist, um:
auf der Grundlage einer Eingabe eine erste Darstellung des biologischen Gewebes (12) zu erzeugen oder diese als Eingabe zu empfangen, die einen tatsächlichen Zustand des biologischen Gewebes (12) nach Durchführung der mindestens einen Arbeitshandlung daran umfasst;
eine zweite Darstellung des biologischen Gewebes zu erzeugen, die einen geplanten Zustand des biologischen Gewebes (12) nach der mindestens einen Arbeitshandlung umfasst; und
eine Bewertung darüber vorzunehmen, inwieweit die Maßnahme erfolgreich durchgeführt wurde, indem die erste Darstellung des biologischen Gewebes (12) mit der zweiten Darstellung des biologischen Gewebes (12) verglichen wird, und die Bewertung bei der Entscheidungsfindung zu nutzen; und
wobei das den Laser umfassende Werkzeug (24) ferner eine Sprüheinrichtung (157) zum Sprühen einer Flüssigkeit auf das biologische Gewebe (12) umfasst, um die Laserablation zu unterstützen und das biologische Gewebe (12) zumindest bis zu einem gewissen Grad vor thermischen Schäden zu schützen.

2. System nach Anspruch 1, wobei die Eingabeeinrichtung (22) mindestens ein Sensorsystem umfasst, das einen Satz von Sensoren (101), (102), (170), (180) umfasst, wobei einzelne Sensoren innerhalb des Sensorsatzes dazu ausgelegt sind, Sensordaten und/oder Informationen zu überwachen, zu erfassen, zu sammeln oder zu messen, die mit einer oder mehreren Eigenschaften, Merkmalen und/oder Parametern eines oder mehrerer des Systems (10), des biologischen Gewebes, der Umgebung, von Komponenten oder damit verbundenen oder daran gekoppelten Systemen oder Vorrichtungen in Zusammenhang stehen oder sich darauf beziehen, wobei die Sensoren (101), (102), (170), (180) des Satzes von Sensoren solche umfassen, die auf einem oder mehreren basieren von: Raman-Spektroskopie; hyperspektrale Bildgebung; optische Bildgebung; Wärmebildgebung; Fluoreszenzspektroskopie, Mikroskopie, Akustik, Messtechnik, optische Kohärenztomographie, Laserleistung und jegliche nicht-invasive Erfassung.

3. System nach Anspruch 1, wobei die vom System (10) durchgeführten Vorgänge entweder halbautomatisch unter der Kontrolle eines Chirurgen oder automatisch ohne menschliches Eingreifen erfolgen.

4. System nach Anspruch 1, wobei die Verarbeitung der Eingabe die Optimierung des Laserbetriebs auf der Grundlage der Wechselwirkungsdynamik zwischen einem vom Laser erzeugten Strahlungsstrahl (30) und dem biologischen Gewebe (12) umfasst, um die Ablationsrate zu erhöhen.

5. System nach Anspruch 1, wobei Strahlungsstrahlen (30) vom Laser in Impulsen erzeugt werden und die Laserimpulse zu Ablationsdurchläufen zusammengefasst werden, die eine vorab berechnete Folge von Impulsen an verschiedenen Stellen über das biologische Gewebe umfassen.

6. System nach Anspruch 1, wobei das System (10) eine Kühleinrichtung (140) zum Kühlen einer oder mehrerer Komponenten und Flüssigkeiten des Systems (10) umfasst.

7. System nach Anspruch 1, umfassend eine Abschirmung zum Schutz während der Bearbeitung des biologischen Gewebes.

8. System nach Anspruch 1, wobei die Positioniereinrichtung (25) einen Roboterarm umfasst.

9. System nach Anspruch 1, wobei, wenn die Eingabe eine Eingabe von einem 3D-Messsensor umfasst, die Verarbeitung die Verwendung der Eingabe zum Abbilden der Geometrie des biologischen Gewebes (12) und der umliegenden Umgebung umfasst.

10. System nach Anspruch 1, wobei, wenn die Eingabe eine Eingabe von einem akustischen Sensor umfasst, die Verarbeitung die Verwendung der Eingabe des akustischen Sensors umfasst, um zu erkennen, ob das Werkzeug (24) unbeabsichtigtes oder unerwartetes biologisches Gewebe (12) oder Material bearbeitet hat.

11. System nach Anspruch 1, wobei der Controller (100) einen intelligenten Controller umfasst, die Software für künstliche Intelligenz (KI) enthält, einschließlich, aber nicht beschränkt auf maschinelle Wahrnehmung oder maschinelles Lernen.

12. System nach Anspruch 1, das ferner eine dynamisch fokussierende Optik und einen Controller für die dynamisch fokussierende Optik umfasst; wobei die dynamisch fokussierende Optik so betreibbar ist, dass sie die Brennweite und den Fokusdurchmesser des Laserstrahls (30) bei einer Zieldistanz dynamisch verändert, der von dem Controller über den Controller für die dynamisch fokussierende Optik als wünschenswert bestimmt wird.

13. Computerlesbares Speichermedium, auf dem Befehle gespeichert sind, die, wenn sie von einer Rechnereinrichtung ausgeführt werden, bewirken, dass die Rechnereinrichtung ein Verfahren durchführt, wobei das Verfahren umfasst:
Speichern elektronischer Programmbefehle zur Steuerung eines Controllers (100); und
Steuern des Controllers (100) über die elektronischen Programmbefehle zum:
Empfangen von Eingaben über eine Eingabeeinrichtung (22); und
Verarbeiten der Eingabe und, auf der Grundlage der Verarbeitung, Steuern eines Werkzeugs (24), das zur Ausführung mindestens einer Arbeitshandlung betreibbar ist, sowie von einer Positioniereinrichtung zum Positionieren des Werkzeugs (24) relativ zum biologischen Gewebe (12), um die mindestens eine Arbeitshandlung auszuführen, um das biologische Gewebe (12) zu bearbeiten,
wobei die von dem Controller (100) durchgeführte Verarbeitung der Eingabe eine Analyse der Eingabe und eine Entscheidungsfindung auf der Grundlage der Analyse sowie von Kriterien umfasst, die von dem Controller (100) empfangen werden, wobei es sich bei den Kriterien um Kriterien handelt, die für die mindestens eine Arbeitshandlung relevant sind, um die Positioniereinrichtung (25) und das Werkzeug (24) zur Bearbeitung des biologischen Gewebes (12) anzusteuern;
und, sobald eine Entscheidung getroffen wurde, das Verfahren das Anweisen des Controllers (100) über die elektronischen Programmbefehle umfasst, auf der Grundlage der Entscheidung eine Aktion einzuleiten, um die Positioniereinrichtung (25) und das Werkzeug (24) zur Bearbeitung des biologischen Gewebes zu steuern; wobei die Kriterien umfassen: Geschwindigkeit der mindestens einen Arbeitshandlung; Genauigkeit der mindestens einen Arbeitshandlung; Sicherheit der mindestens einen Arbeitshandlung; und Sauberkeit der mindestens einen Arbeitshandlung; und, wobei der Controller (100) als zumindest Teil der Analyse unter der Steuerung der elektronischen Programmbefehle betreibbar ist, um:
auf der Grundlage einer Eingabe eine erste Darstellung des biologischen Gewebes (12) zu erzeugen oder als Eingabe zu empfangen, wobei diese Darstellung einen tatsächlichen Zustand des biologischen Gewebes (12) umfasst, nachdem mindestens eine Arbeitshandlung daran durchgeführt wurde;
auf der Grundlage einer Eingabe eine zweite Darstellung des biologischen Gewebes (12) zu erzeugen oder als Eingabe zu empfangen, wobei die andere Darstellung des biologischen Gewebes (12) eine andere Darstellung des biologischen Gewebes (12) ist und einen geplanten Zustand des biologischen Gewebes (12) nach der mindestens einen Arbeitshandlung umfasst; und
eine Bewertung darüber vorzunehmen, inwieweit der Eingriff erfolgreich durchgeführt wurde, indem die erste Darstellung des biologischen Gewebes (12) mit der zweiten Darstellung des biologischen Gewebes (12) verglichen wird, und die Bewertung bei der Entscheidungsfindung heranzuziehen;
wobei das Werkzeug (24) ferner eine Sprüheinrichtung (157) zum Sprühen einer Flüssigkeit auf das biologische Gewebe (12) umfasst, um die Laserablation zu unterstützen und das biologische Gewebe (12) zumindest bis zu einem gewissen Grad vor thermischen Schäden zu schützen.

## Revendications

1. Système d'ostéotomie au laser assisté par robot (10) destiné à modeler un tissu biologique (12), y compris un os, le système (10) comprenant :
un outil (24) comprenant un laser capable d'effectuer au moins une action de travail comprenant une ablation au laser ;
un moyen de positionnement (25) destiné à positionner l'outil (24) par rapport au tissu biologique (12) afin d'effectuer ladite au moins une action de travail ;
un moyen d'entrée (22) ;
un contrôleur (100) destiné à recevoir des critères en entrée via le moyen d'entrée (22), à les traiter et, sur la base de ce traitement, à commander le moyen de positionnement (25) et l'outil (24) pour travailler le tissu biologique (12) ; et
un stockage (18) stockant des instructions de programme électroniques pour le fonctionnement du contrôleur (100) ;
dans lequel le traitement de l'entrée effectué par le contrôleur (100), sous l'action des instructions de programme électroniques, comprend une analyse de l'entrée et une prise de décision sur la base de cette analyse et des critères reçus par le contrôleur (100), lesdits critères étant des critères pertinents pour ladite au moins une action de travail afin de diriger le moyen de positionnement (25) et l'outil (24) pour travailler le tissu biologique (12) ; et, une fois qu'une décision a été prise, le contrôleur est configuré, sous l'action des instructions de programme électroniques, pour déclencher une action sur la base de la décision afin de commander le moyens de positionnement et l'outil pour travailler le matériau ;
dans lequel les critères comprennent : la vitesse de ladite au moins une action de travail ; la précision de ladite au moins une action de travail ; la sécurité de ladite au moins une action de travail ; et la propreté de ladite au moins une action de travail ;
dans lequel, dans le cadre d'au moins une partie de l'analyse, le contrôleur (100) est apte à :
générer, sur la base d'une entrée, ou recevoir en entrée, une première représentation du tissu biologique (12) comprenant un état réel du tissu biologique (12) après que ladite au moins une action de travail a été effectuée sur celui-ci ;
générer une deuxième représentation du tissu biologique comprenant un état prévu du tissu biologique (12) après ladite au moins une action de travail ; et
évaluer dans quelle mesure l'action a été menée à bien en comparant la première représentation du tissu biologique (12) à la deuxième représentation du tissu biologique (12) et utiliser cette évaluation pour prendre une décision ; et
dans lequel l'outil (24) comprenant le laser inclut en outre un pulvérisateur (157) destiné à pulvériser un liquide sur le tissu biologique (12) afin de faciliter l'ablation au laser et de protéger le tissu biologique (12) contre un dommage thermique, au moins dans une certaine mesure.

2. Système selon la revendication 1, dans lequel le moyen d'entrée (22) comprend au moins un système de capteurs comprenant un ensemble de capteurs (101), (102), (170), (180), dans lequel des capteurs individuels de l'ensemble de capteurs sont aptes à surveiller, détecter, collecter ou mesurer des données de capteurs et/ou des informations associées ou relatives à une ou plusieurs caractéristiques, propriétés et/ou paramètres d'un ou plusieurs éléments parmi le système (10), le tissu biologique, l'environnement environnant, des composants, ou des systèmes ou dispositifs qui y sont associés ou couplés, dans lequel les capteurs (101), (102), (170), (180) de l'ensemble de capteurs incluent ceux basés sur un ou plusieurs des principes suivants : la spectroscopie Raman ; l'imagerie hyperspec-trale ; l'imagerie optique ; l'imagerie thermique ; la spectroscopie de fluorescence, la microscopie, l'acoustique, la métrologie, la tomographie par cohérence optique, la puissance laser, et toute détection non invasive.

3. Système selon la revendication 1, dans lequel les opérations effectuées par le système (10) se déroulent soit de manière semi-automatique sous contrôle d'un chirurgien, soit de manière automatique sans nécessiter d'intervention humaine.

4. Système selon la revendication 1, dans lequel le traitement de l'entrée comprend l'optimisation du fonctionnement du laser en fonction de la dynamique d'interaction entre un faisceau (30) de rayonnement généré par le laser et le tissu biologique (12) afin d'augmenter la vitesse d'ablation.

5. Système selon la revendication 1, dans lequel des faisceaux (30) de rayonnement sont générés par le laser sous forme d'impulsions, et les impulsions laser sont regroupées en séries d'ablation comprenant un ensemble précal-culé d'impulsions à différents emplacements à travers le tissu biologique.

6. Système selon la revendication 1, dans lequel le système (10) inclut un moyen de refroidissement (140) destiné à refroidir un ou plusieurs composants et liquides du système (10).

7. Système selon la revendication 1, comprenant un blindage destiné à assurer une protection pendant le travail du tissu biologique.

8. Système selon la revendication 1, dans lequel le moyen de positionnement (25) comprend un bras robotisé.

9. Système selon la revendication 1, dans lequel, lorsque l'entrée comprend une entrée provenant d'un capteur de métrologie 3D, le traitement comprend d'utiliser l'entrée pour cartographier la géométrie du tissu biologique (12) et de l'environnement environnant.

10. Système selon la revendication 1, dans lequel, lorsque l'entrée comprend une entrée provenant d'un capteur acoustique, le traitement comprend d'utiliser l'entrée du capteur acoustique pour détecter si l'outil (24) a agi sur un tissu biologique (12) ou matériau non visé ou inattendu.

11. Système selon la revendication 1, dans lequel le contrôleur (100) comprend un contrôleur intelligent doté d'un logiciel d'intelligence artificielle (IA) incluant, sans s'y limiter, la perception automatique ou l'apprentissage automatique.

12. Système selon la revendication 1, comprenant en outre une optique à mise au point dynamique et un contrôleur d'optique à mise au point dynamique ; l'optique à mise au point dynamique étant apte à modifier de manière dynamique la distance focale et le diamètre de faisceau laser du faisceau laser (30) à une distance cible déterminée comme souhaitable par le contrôleur via le contrôleur d'optique à mise au point dynamique.

13. Support de stockage lisible par ordinateur sur lequel sont stockées des instructions qui, lorsqu'elles sont exécutées par un moyen de calcul, amènent le moyen de calcul à mettre en œuvre un procédé, le procédé comprenant :
le stockage d'instructions de programme électroniques destinées à commander un contrôleur (100) ; et
la commande du contrôleur (100) via les instructions de programme électroniques, afin de :
recevoir une entrée via un moyen d'entrée (22) ; et
traiter l'entrée et, sur la base de ce traitement, commander un outil (24) apte à effectuer au moins une action de travail, ainsi qu'un moyen de positionnement destiné à positionner l'outil (24) par rapport au tissu biologique (12) afin d'effectuer ladite au moins une action de travail, pour travailler le tissu biologique (12),
dans lequel : le traitement de l'entrée effectué par le contrôleur (100) comprend une analyse de l'entrée et une prise de décision sur la base de cette analyse et de critères reçus par le contrôleur (100), lesdits critères étant des critères pertinents pour ladite au moins une action de travail afin de diriger le moyen de positionnement (25) et l'outil (24) pour travailler le tissu biologique (12) ; et
une fois la décision prise, le procédé comprend la commande du contrôleur (100), via les instructions de programme électroniques, pour déclencher une action sur la base de la décision, afin de commander le moyen de positionnement (25) et l'outil (24) pour travailler le tissu biologique ; dans lequel les critères comprennent : la vitesse de ladite au moins une action de travail ; la précision de ladite au moins une action de travail ; la sécurité de ladite au moins une action de travail ; et la propreté de ladite au moins une action de travail ; et dans lequel
dans le cadre d'au moins une partie de l'analyse, le contrôleur (100) est apte, sous l'action des instructions de programme électroniques, à :
générer, à partir d'une entrée, ou recevoir en entrée, une première représentation du tissu biologique (12) comprenant un état réel dudit tissu biologique (12) après que ladite au moins une action de travail a été effectuée sur celui-ci ;
générer, sur la base d'une entrée, ou recevoir en entrée, une deuxième représentation du tissu biologique (12), cette autre représentation du tissu biologique (12) étant une représentation différente du tissu biologique (12) et comprenant un état prévu du tissu biologique (12) après la réalisation de ladite au moins une action de travail ; et
évaluer dans quelle mesure l'action a été menée à bien en comparant la première représentation du tissu biologique (12) à la deuxième représentation du tissu biologique (12) et utiliser cette évaluation pour prendre la décision ;
dans lequel l'outil (24) inclut en outre un pulvérisateur (157) destiné à pulvériser un liquide sur le tissu biologique (12) afin de faciliter l'ablation au laser et de protéger le tissu biologique (12) contre un dommage thermique, au moins dans une certaine mesure.
